(19) Europäisches Patentamt
European Patent Office
Office européen des brevets

(11) **EP 4 570 322 A1**

(12) **EUROPEAN PATENT APPLICATION**
published in accordance with Art. 153(4) EPC

(43) Date of publication:
**18.06.2025 Bulletin 2025/25**

(21) Application number: **23852562.0**

(22) Date of filing: **08.08.2023**

(51) International Patent Classification (IPC):
***A61Q 1/00*** (2006.01) ***A61Q 19/00*** (2006.01)
***A61K 8/06*** (2006.01) ***A61K 8/31*** (2006.01)
***A61K 8/34*** (2006.01) ***A61K 8/36*** (2006.01)
***A61K 8/37*** (2006.01) ***A61K 8/44*** (2006.01)
***A61K 8/67*** (2006.01) ***A61K 8/68*** (2006.01)
***A61K 8/81*** (2006.01)

(52) Cooperative Patent Classification (CPC):
**A61K 8/06; A61K 8/31; A61K 8/34; A61K 8/36; A61K 8/37; A61K 8/40; A61K 8/42; A61K 8/44; A61K 8/46; A61K 8/67; A61K 8/68; A61K 8/81; A61Q 1/00; A61Q 19/00**

(86) International application number:
**PCT/JP2023/028895**

(87) International publication number:
**WO 2024/034600 (15.02.2024 Gazette 2024/07)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **08.08.2022 JP 2022126617**

(71) Applicant: **Kao Corporation**
**Tokyo 103-8210 (JP)**

(72) Inventor: **TAIMA, Hidetoshi**
**Odawara-shi, Kanagawa 250-0002 (JP)**

(74) Representative: **Hoffmann Eitle**
**Patent- und Rechtsanwälte PartmbB**
**Arabellastraße 30**
**81925 München (DE)**

(54) **OIL-IN-WATER-TYPE EMULSION COMPOSITION**

(57) Provided is an oil-in-water-type emulsion composition comprising the following components (A), (B), (C), (D), and (E): (A) a hydrocarbon oil which is in a liquid state at 25°C, (B) one or more members selected from the group consisting of a higher fatty acid, a higher alcohol, and an ester oil which are in a liquid state at 25°C, (C) from 0.1 to 5% by mass of one or more members selected from the group consisting of a cationic surfactant and an amphoteric surfactant, (D) from 2 to 20% by mass of one or more members selected from the group consisting of a ceramide, a hydrocarbon oil which is in a paste state at 25°C, and an ester oil which is in a paste state at 25°C, and (E) water, wherein a total content of the components (A) and (B), (A) + (B), is from 1 to 30% by mass, and a mass ratio of the component (B) to the total amount of the components (A) and (B), (B) / ((A) + (B)), is from 0.05 to 0.9.

EP 4 570 322 A1

## Description

### Field of the Invention

The present invention relates to an oil-in-water-type emulsion composition.

### Background of the Invention

Heretofore, moisturizing care throughout the body has been performed, for example, by the application of cosmetics having a moisturizing effect in a dry state of the body immediately after bathing to bedtime.

For example, a ceramide dispersion composition containing natural ceramide, a polyhydric alcohol, a nonionic surfactant, an amphoteric surfactant, and a cationic surfactant (Patent Literature 1), and an emulsion composition containing a compound selected from the group consisting of sphingosine, pseudo-sphingosine, their salts, an anionic surfactant, and a cationic surfactant, a ceramide, glycerin mono-fatty acid ester, a higher alcohol, and water (Patent Literature 2) have been studied as compositions containing ceramides or the like having a high moisturizing effect.

(Patent Literature 1) JP-A-2015-30705
(Patent Literature 2) JP-A-2007-22997

### Summary of the Invention

The present invention relates to an oil-in-water-type emulsion composition comprising the following components (A), (B), (C), (D), and (E):

(A) a hydrocarbon oil which is in a liquid state at 25°C,
(B) one or more members selected from the group consisting of a higher fatty acid, a higher alcohol, and an ester oil which are in a liquid state at 25°C,
(C) from 0.1 to 5% by mass of one or more members selected from the group consisting of a cationic surfactant and an amphoteric surfactant,
(D) from 2 to 20% by mass of one or more members selected from the group consisting of a ceramide, a hydrocarbon oil which is in a paste state at 25°C, and an ester oil which is in a paste state at 25°C, and
(E) water, wherein

a total content of the components (A) and (B), (A) + (B), is from 1 to 30% by mass, and
a mass ratio of the component (B) to the total amount of the components (A) and (B), (B) / ((A) + (B)), is from 0.05 to 0.9.

The present invention also relates to an oil-in-water-type emulsion composition comprising the following components (A), (B), (C), (D), and (E):

(A) a hydrocarbon oil which is in a liquid state at 25°C,
(B) one or more members selected from the group consisting of a higher fatty acid, a higher alcohol, and an ester oil which are in a liquid state at 25°C,
(C) from 0.1 to 5% by mass of a cationic surfactant,
(D) from 1 to 20% by mass of a ceramide, and
(E) water.

### Brief Description of the Drawing

Fig... 1 illustrates a method for collecting a site where an oil-in-water-type emulsion composition was applied in Test Example.

### Detailed Description of the Invention

Even if cosmetics or the like are applied to the skin throughout the body after bathing, their effects are difficult to sufficiently sustain or improve for persons having dry sensitive skin, disadvantageously resulting in repetitive dryness and roughness of the skin throughout the body. For example, cosmetics, after application, may run down upon sweating or

when splashed with water so that their moisturizing effects are impaired. When wet skin is toweled after bathing and then a cosmetic is applied to the skin throughout the body, the toweling takes time, and it also takes time to spread the cosmetic over the dried-off skin because the cosmetic is difficult to spread. Hence, the time required for toweling wet skin and the time required for spreading the cosmetic over the skin throughout the body are combined while the skin disadvantageously starts to dry.

Additionally, cosmetics having a high moisturizing effect tend to cause a sticky or slimy feeling.

Stickiness after cosmetic application may be suppressed. However, persons who are still uncomfortable with cosmetics applied to the skin want to wash off the cosmetics, for example, by taking a shower, and however, disadvantageously cannot obtain an effect such as a moist feeling or a glow feeling after application because the cosmetics run down by washing.

The present inventor found that an oil-in-water-type emulsion composition containing two or more specific liquid oils at a specific ratio and further containing one or more members selected from the group consisting of a cationic surfactant and an amphoteric surfactant, and one or more members selected from the group consisting of a ceramide, a hydrocarbon oil which is in a paste state at 25°C, and an ester oil which is in a paste state at 25°C, is excellent in an absorption feeling upon application of the oil-in-water-type emulsion composition to dry skin and a moist feeling after subsequent washing off with water, is easy to spread without stickiness or sliminess and produces a cover or protection feeling when applied to the skin wetted with water, and is excellent in a skin glow feeling without stickiness even if applied to wet skin and washed off with water.

The oil-in-water-type emulsion composition of the present invention, when applied to the skin wetted with water, produces a cover or protection feeling because the oil-in-water-type emulsion composition remains on the skin even if toweled.

The component (A) used in the present invention is a hydrocarbon oil which is in a liquid state at 25°C.

In the present invention, the liquid state refers to a state in which a viscosity at 25°C is 10 000 mPa·s or less. The viscosity is measured at 12 rpm for 1 minute with a type B viscometer (VISCOMETER TVB-10, manufactured by Toki Sangyo Co., Ltd.) with rotor No. 4.

Examples of the hydrocarbon oil as the component (A) include squalane, squalene, liquid paraffin, liquid isoparaffin, light isoparaffin, light liquid isoparaffin, heavy liquid isoparaffin, $\alpha$-olefin oligomers, and isododecane.

The hydrocarbon oil preferably comprises one or more members selected from the group consisting of squalane, liquid paraffin, liquid isoparaffin, light liquid isoparaffin, light isoparaffin, and an $\alpha$-olefin oligomer, more preferably comprises one or more members selected from the group consisting of squalane, liquid paraffin, liquid isoparaffin, and an $\alpha$-olefin oligomer, further more preferably comprises one or more members selected from the group consisting of liquid paraffin, liquid isoparaffin, and an $\alpha$-olefin oligomer, and even more preferably comprises liquid paraffin.

One or more components (A) can be used. A content thereof is preferably 0.1% by mass or more, more preferably 1% by mass or more, further more preferably 2% by mass or more, even more preferably 4% by mass or more, and preferably 20% by mass or less, more preferably 10% by mass or less, further more preferably 7% by mass or less, even more preferably 6.5% by mass or less, particularly preferably 6% by mass or less, in the total composition from the viewpoint of improving stability, an absorption feeling upon application to dry skin, a moist feeling after washing with water, easy spreading upon application to wet skin, the absence of sliminess upon application, a cover or protection feeling, next morning's moist feeling, a skin glow feeling after application to the skin and washing off with water, and the absence of stickiness. The content of the component (A) is preferably from 0.1 to 20% by mass, more preferably from 1 to 10% by mass, further more preferably from 1 to 7% by mass, even more preferably from 2 to 6.5% by mass, particularly preferably from 4 to 6% by mass, in the total composition.

The component (B) is one or more members selected from the group consisting of a higher fatty acid, a higher alcohol, and an ester oil which are in a liquid state at 25°C.

Examples of the higher fatty acid as the component (B) include oleic acid, isostearic acid, and undecylenic acid. Among them, the higher fatty acid preferably comprises one or more members selected from the group consisting of oleic acid and isostearic acid from the viewpoint of improving a moist feeling after application to dry skin and then washing with water, a cover or protection feeling upon application to wet skin, next morning's moist feeling, and a skin glow feeling after application to the skin and washing off with water.

Examples of the higher alcohol include octyl alcohol, decyl alcohol, 2-octyldodecanol, hexyldecanol, isostearyl alcohol, and/or oleyl alcohol. Among them, the higher alcohol is preferably 2-octyldodecanol, isostearyl alcohol, or oleyl alcohol and more preferably comprises one or more members selected from the group consisting of isostearyl alcohol and oleyl alcohol, from the viewpoint of improving a moist feeling after application to dry skin and then washing with water, a cover or protection feeling upon application to wet skin, next morning's moist feeling, and a skin glow feeling after application to the skin and washing off with water.

Examples of the ester oil include monoester oil, diester oil, triester oil, and tetraester oil. Any ester oil may be used as long as it is in a liquid state at 25°C.

Examples of the monoester oil include monoester of aliphatic or aromatic monocarboxylic acid or dicarboxylic

acid having 2 to 24 carbon atoms. Specific examples thereof include cetyl 2-ethylhexanoate, cetyl octanoate, isononyl isononanoate, isotridecyl isononanoate, hexyl laurate, isopropyl myristate, octyldodecyl myristate, myristyl myristate, 2-hexyldecyl myristate, isopropyl palmitate, octyl palmitate, 2-hexyldecyl palmitate butyl stearate, isocetyl stearate, isocetyl isostearate, decyl oleate, isodecyl benzoate, octyl methoxycinnamate, hexyldecyl dimethyloctanoate, cetyl lactate, myristyl lactate, 2-ethylhexyl succinate, 2-hexyldecyl adipate, and alkyl (C12 to C15) benzoate.

The monoester oil preferably comprises one or more members selected from the group consisting of isononyl isononanoate, isotridecyl isononanoate, and octyldodecyl myristate and more preferably comprises isononyl isononanoate, from the viewpoint of improving stability of the oil-in-water-type emulsion composition, an absorption feeling upon application to dry skin, a moist feeling after washing with water, easy spreading upon application to wet skin, the absence of sliminess upon application, a cover or protection feeling, next morning's moist feeling, a skin glow feeling after application to the skin and washing off with water, the absence of stickiness.

Examples of the diester oil include diester of dicarboxylic acid having 3 to 18 carbon atoms and difatty acid ester of a polyhydric alcohol. Specific examples thereof include propylene glycol dicaprylate, neopentyl glycol dicaprate, glycol distearate, propylene glycol diisostearate, glyceryl diisostearate, glyceryl monoisostearate monomyristate, glycerin di-2-heptylundecanoate, di-2-ethylhexyl succinate, diisopropyl sebacate, diisostearyl malate, ethylene glycol di-2-ethylhexanoate, diisobutyl adipate, di-2-heptylundecyl adipate, and di-2-ethylhexyl sebacate. Examples of the acylamino acid ester include acylamino acid diester such as di(cholesteryl/2-octyldodecyl) N-lauroyl-L-glutamate and di(phytosteryl/2-octyldodecyl) N-lauroyl-L-glutamate.

The diester oil preferably comprises one or more members selected from the group consisting of propylene glycol dicaprylate, neopentyl glycol dicaprate, di(cholesteryl/2-octyldodecyl) N-lauroyl-L-glutamate, and di(phytosteryl/2-octyldodecyl) N-lauroyl-L-glutamate, more preferably comprises one or more members selected from the group consisting of neopentyl glycol dicaprate, di(cholesteryl/2-octyldodecyl) N-lauroyl-L-glutamate, and di(phytosteryl/2-octyldodecyl) N-lauroyl-L-glutamate, further more preferably comprises one or more members selected from the group consisting of neopentyl glycol dicaprate and di(phytosteryl/2-octyldodecyl) N-lauroyl-L-glutamate, and even more preferably comprises di(phytosteryl/2-octyldodecyl) lauroyl-L-glutamate, from the viewpoint of improving stability of the oil-in-water-type emulsion composition, an absorption feeling upon application to dry skin, a moist feeling after washing with water, easy spreading upon application to wet skin, the absence of sliminess upon application, a cover or protection feeling, next morning's moist feeling, a skin glow feeling after application to the skin and washing off with water, and the absence of stickiness. The diester oil is preferably acylamino acid ester.

Examples of the triester oil include triglycerin fatty acid ester. One or more constituent fatty acids are preferably fatty acid triglyceride having 6 to 22 carbon atoms. Two or more constituent fatty acids more preferably have 6 to 22 carbon atoms, and all constituent fatty acids further more preferably have 6 to 22 carbon atoms, from the viewpoint of improving stability of the oil-in-water-type emulsion composition, an absorption feeling upon application to dry skin, a moist feeling after washing with water, easy spreading upon application to wet skin, the absence of sliminess upon application, a cover or protection feeling, next morning's moist feeling, a skin glow feeling after application to the skin and washing off with water, and the absence of stickiness.

Examples of such triglycerin fatty acid ester include caprylic/capric triglyceride, caprylic/capric/myristic/stearic triglyceride, glyceryl tri-2-ethylhexanoate, glyceryl tricaprylate, glyceryl triethylhexanoate, glyceryl tristearate, glyceryl triisostearate, glyceryl tripalmitate, glyceryl tri-2-heptylundecanoate, glyceryl tribehenate, glyceryl trimyristate, glyceryl trilaurate, glyceryl trioleate, glyceryl trilinoleate, and glyceryl tripalmitoleate. Further examples thereof include plant oils such as tricoconut oil fatty acid glyceryl, olive oil, jojoba oil, macadamia nut oil, meadowfoam oil, castor oil, safflower oil, sunflower oil, avocado oil, canola oil, apricot kernel oil, rice germ oil, and rice bran oil.

Among them, the triglycerin fatty acid ester preferably comprises one or more members selected from the group consisting of caprylic/capric triglyceride, caprylic/capric/myristic/stearic triglyceride, glyceryl tri-2-ethylhexanoate, glyceryl tricaprylate, glyceryl triethylhexanoate, glyceryl tristearate, glyceryl triisostearate, glyceryl tripalmitate, glyceryl trioleate, and a plant oil, more preferably comprises one or more members selected from the group consisting of caprylic/capric triglyceride, glyceryl tri-2-ethylhexanoate, and a plant oil, further more preferably comprises one or more members selected from the group consisting of caprylic/capric triglyceride, glyceryl tri-2-ethylhexanoate, olive oil, and jojoba oil, even more preferably comprises one or more members selected from the group consisting of caprylic/capric triglyceride and olive oil, and particularly preferably comprises caprylic/capric triglyceride, from the viewpoint of improving stability of the oil-in-water-type emulsion composition, an absorption feeling upon application to dry skin, a moist feeling after washing with water, easy spreading upon application to wet skin, the absence of sliminess upon application, a cover or protection feeling, next morning's moist feeling, a skin glow feeling after application to the skin and washing off with water, and the absence of stickiness.

Examples of the tetraester oil include tetra-fatty acid ester of a tetrahydric or higher polyhydric alcohol and specifically include pentaerythritol tetra(behenate/benzoate/ethylhexanoate), pentaerythritol tetraethylhexanoate, pentaerythritol tetraoctanoate, and pentaerythritol tetra-2-ethylhexanoate.

The component (B) preferably comprises one or more members selected from the group consisting of a

monoester oil, a diester oil, and a triester oil, more preferably comprises a diester oil, and further more preferably comprises acylamino acid diester, from the viewpoint of improving stability of the oil-in-water-type emulsion composition, an absorption feeling upon application to dry skin, a moist feeling after washing with water, easy spreading upon application to wet skin, the absence of sliminess upon application, a cover or protection feeling, next morning's moist feeling, a skin glow feeling after application to the skin and washing off with water, and the absence of stickiness.

The component (B) preferably comprises one or more members selected from the group consisting of di(phytosteryl/2-octyldodecyl) N-lauroyl-L-glutamate, neopentyl glycol dicaprate, isononyl isononanoate, and caprylic/-capric triglyceride and more preferably comprises di(phytosteryl/2-octyldodecyl) N-lauroyl-L-glutamate, from the viewpoint of improving stability of the oil-in-water-type emulsion composition, an absorption feeling upon application to dry skin, a moist feeling after washing with water, easy spreading upon application to wet skin, the absence of sliminess upon application, a cover or protection feeling, next morning's moist feeling, a skin glow feeling after application to the skin and washing off with water, and the absence of stickiness.

One or more components (B) can be used in combination. A content thereof is preferably 0.5% by mass or more, more preferably 1% by mass or more, further more preferably 1.5% by mass or more, even more preferably 2% by mass or more, and preferably 10% by mass or less, more preferably 8% by mass or less, further more preferably 6% by mass or less, even more preferably 4% by mass or less, in the total composition from the viewpoint of improving stability of the oil-in-water-type emulsion composition, an absorption feeling upon application to dry skin, a moist feeling after washing with water, easy spreading upon application to wet skin, the absence of sliminess upon application, a cover or protection feeling, next morning's moist feeling, a skin glow feeling after application to the skin and washing off with water, and the absence of stickiness. The content of the component (B) is preferably from 0.5 to 10% by mass, more preferably from 1 to 8% by mass, further more preferably from 1.5 to 6% by mass, even more preferably from 2 to 4% by mass, in the total composition.

In the present invention, a mass ratio of the component (A) to the component (B), (A) / (B), is preferably 0.1 or more, more preferably 0.45 or more, further more preferably 1.1 or more, and preferably 10 or less, more preferably 7 or less, further more preferably 3 or less, from the viewpoint of improving stability, an absorption feeling upon application to dry skin, a moist feeling after washing with water, easy spreading upon application to wet skin, the absence of sliminess upon application, a cover or protection feeling, next morning's moist feeling, a skin glow feeling after application to the skin and washing off with water, and the absence of stickiness. The mass ratio of the component (A) to the component (B), (A) / (B), is preferably from 0.1 to 10, more preferably from 0.45 to 7, further more preferably from 1.1 to 3.

In the present invention, a total content of the components (A) and (B), (A) + (B), is preferably 1% by mass or more, more preferably 2% by mass or more, further more preferably 3% by mass or more, even more preferably 5% by mass or more, and preferably 30% by mass or less, more preferably 20% by mass or less, further more preferably 15% by mass or less, even more preferably 10% by mass or less, in the total composition from the viewpoint of improving stability of the oil-in-water-type emulsion composition, an absorption feeling upon application to dry skin, a moist feeling after washing with water, easy spreading upon application to wet skin, the absence of sliminess upon application, a cover or protection feeling, next morning's moist feeling, a skin glow feeling after application to the skin and washing off with water, and the absence of stickiness. The total content of the components (A) and (B), (A) + (B), is preferably from 1 to 30% by mass, more preferably from 2 to 20% by mass, further more preferably from 3 to 15% by mass, even more preferably from 5 to 10% by mass, in the total composition.

In the present invention, a mass ratio of the component (B) to the total amount of the components (A) and (B), (B) / ((A) + (B)), is 0.05 or more, preferably 0.1 or more, more preferably 0.15 or more, further more preferably 0.3 or more, even more preferably 0.35 or more, and 0.9 or less, preferably 0.8 or less, more preferably 0.75 or less, further more preferably 0.7 or less, even more preferably 0.5 or less, from the viewpoint of improving stability of the oil-in-water-type emulsion composition, an absorption feeling upon application to dry skin, a moist feeling after washing with water, easy spreading upon application to wet skin, the absence of sliminess upon application, a cover or protection feeling, next morning's moist feeling, a skin glow feeling after application to the skin and washing off with water, and the absence of stickiness. The mass ratio of the component (B) to the total amount of the components (A) and (B), (B) / ((A) + (B)), is from 0.05 to 0.9, preferably from 0.1 to 0.8, more preferably from 0.15 to 0.75, further more preferably from 0.3 to 0.7, even more preferably from 0.35 to 0.5.

The component (C) is one or more members selected from the group consisting of a cationic surfactant and an amphoteric surfactant.

Any cationic surfactant may be used as the component (C) as long as it can be used in usual cosmetics. Examples thereof include quaternary ammonium salt-type cationic surfactants and amine salt-type cationic surfactants. A quaternary ammonium salt-type cationic surfactant is preferred. Examples of the amine salt-type cationic surfactant include alkylamine salts.

Such a cationic surfactant is preferably a quaternary ammonium salt-type cationic surfactant of the following formula (1):

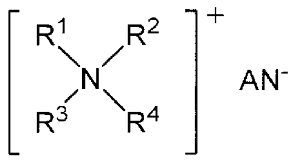

(1)

wherein

$R^1$ represents a linear, branched, or cyclic saturated or unsaturated hydrocarbon group having 6 or more carbon atoms,
$R^2$ represents an alkyl group having 1 to 4 carbon atoms,
$R^3$ and $R^4$ each independently represent an alkyl group having 1 to 4 carbon atoms or a linear, branched, or cyclic saturated or unsaturated hydrocarbon group having 6 or more carbon atoms, and
$AN^-$ represents a salt-forming anion.

In the formula (1), the "linear, branched, or cyclic saturated or unsaturated hydrocarbon group" represented by $R^1$, $R^3$, and $R^4$ preferably has 6 to 30 carbon atoms, more preferably 8 to 24 carbon atoms, further more preferably 12 to 22 carbon atoms, even more preferably 16 to 20 carbon atoms.

Examples of the hydrocarbon group include alkyl groups, alkenyl groups, and aralkyl group. Among them, an alkyl group or an alkenyl group is preferred, and an alkyl group is more preferred.

The alkyl group preferably has 6 to 30 carbon atoms, more preferably 8 to 24 carbon atoms, further more preferably 12 to 22 carbon atoms, even more preferably 16 to 20 carbon atoms, as described above. The alkyl group may be linear or branched. Examples thereof include an octyl group, a nonyl group, a decyl group, an undecyl group, a 1-methyldecyl group, a dodecyl group, a 1-methylundecyl group, a 1-ethyldecyl group, a tridecyl group, a tetradecyl group, a pentadecyl group, a hexadecyl group, an octadecyl group, and a behenyl group.

The alkenyl group may be linear or branched. Examples thereof include a decenyl group, an undecenyl group, a dodecenyl group, a tetradecenyl group, and a hexadecenyl group.

Examples of the aralkyl group include a benzyl group and a phenethyl group.

The "alkyl group having 1 to 4 carbon atoms" represented by $R^2$, $R^3$, and $R^4$ preferably has 1 or 2 carbon atoms, more preferably 1 carbon atom. This alkyl group may be linear or branched. Examples thereof include a methyl group, an ethyl group, a n-propyl group, an isopropyl group, a n-butyl group, and an isobutyl group.

Examples of the combination of $R^3$ and $R^4$ include a combination in which both $R^3$ and $R^4$ are an alkyl group having 1 to 4 carbon atoms, a combination in which one of $R^3$ and $R^4$ is an alkyl group having 1 to 4 carbon atoms and the other is a linear, branched, or cyclic saturated or unsaturated hydrocarbon group having 6 or more carbon atoms, and a combination in which both $R^3$ and $R^4$ are a linear, branched, or cyclic saturated or unsaturated hydrocarbon group having 6 or more carbon atoms. The combination is preferably a combination in which both $R^3$ and $R^4$ are an alkyl group having 1 to 4 carbon atoms or a combination in which one of $R^3$ and $R^4$ is an alkyl group having 1 to 4 carbon atoms and the other is a linear, branched, or cyclic saturated or unsaturated hydrocarbon group having 6 or more carbon atoms, more preferably a combination in which one of $R^3$ and $R^4$ is an alkyl group having 1 to 4 carbon atoms and the other is a linear, branched, or cyclic saturated or unsaturated hydrocarbon group having 6 or more carbon atoms.

In the formula (1), $AN^-$ represents a salt-forming anion. Examples thereof include halide ions (e.g., chloride ions and iodide ions), sulfate ions, phosphate ions, acetate ions, lactate ions, p-toluenesulfonate ions, perchlorate ions, and monoalkyl nitrate ions (e.g., methyl sulfate ions and ethyl sulfate ions). A halide ion is preferred.

Examples of the cationic surfactant include: monoalkyl trimethylammonium salts such as octyl trimethylammonium chloride, decyl trimethylammonium chloride, lauryl trimethylammonium chloride, tetradecyl trimethylammonium chloride, hexadecyl trimethylammonium chloride, hexadecyl trimethylammonium bromide, stearyl trimethylammonium chloride, and behenyl trimethylammonium chloride; dialkyl dimethylammonium salts such as didecyl dimethylammonium chloride and distearyl dimethylammonium chloride; and trialkyl methylammonium salts.

Among them, the cationic surfactant preferably comprises one or more members selected from the group consisting of a monoalkyl trimethylammonium salt and a dialkyl dimethylammonium salt and more preferably comprises a dialkyl dimethylammonium salt. The cationic surfactant preferably comprises one or more members selected from the group consisting of behenyl trimethylammonium chloride and distearyl dimethylammonium chloride and more preferably comprises distearyl dimethylammonium chloride.

Any amphoteric surfactant may be used as the component (C) as long as it can be used in usual cosmetics. Examples thereof include acetic acid betaine-type surfactants such as lauryl dimethylaminoacetic acid betaine, amine oxide-type surfactants such as lauryl dimethylamine oxide, imidazolinium betaine-type surfactants such as 2-alkyl-N-carboxymethyl-N-hydroxyethylimidazolinium betaine, amidobetaine-type surfactants such as lauric acid amidopropyl betaine, sulfobetaine-type surfactants such as lauryl hydroxysulfobetaine, and amino acid-based amphoteric surfactants.

Among them, the amphoteric surfactant preferably comprises one or more members selected from the group consisting of an amino acid-based amphoteric surfactant, a sulfobetaine-type surfactant, an amidobetaine-type surfactant, and an imidazolinium betaine-type surfactant, more preferably comprises one or more members selected from the group consisting of an amino acid-based amphoteric surfactant, a sulfobetaine-type surfactant, and an amidobetaine-type surfactant, further more preferably comprises one or more members selected from the group consisting of an amino acid-based amphoteric surfactant and a sulfobetaine-type surfactant, and even more preferably comprises an amino acid-based surfactant, from the viewpoint of improving emulsifiability and stability of the oil-in-water-type emulsion composition.

For example, a commercially available product such as N-[3-alkyl(12,14)oxy-2-hydroxypropyl]-L-arginine hydrochloride (AMISAFE LMA-60, manufactured by Ajinomoto Co., Inc.), lauryl hydroxysulfobetaine (AMPHITOL 20HD, manufactured by Kao Corp.), or laurylamidopropyl betaine (AMPHITOL 20AB, manufactured by Kao Corp.) can be suitably used as such an amphoteric surfactant. Among them, N-[3-alkyl(12,14)oxy-2-hydroxypropyl]-L-arginine hydrochloride(AMISAFE LMA-60 (manufactured by Ajinomoto Co., Inc.) is preferred.

The component (C) is preferably a cationic surfactant from the viewpoint of improving emulsifiability and stability of the oil-in-water-type emulsion composition and from the viewpoint of improving an absorption feeling upon application to dry skin, a moist feeling after washing with water, easy spreading upon application to wet skin, the absence of sliminess upon application, a cover or protection feeling, next morning's moist feeling, a skin glow feeling after application to the skin and washing off with water, and the absence of stickiness.

One or more components (C) can be used. A content thereof is preferably 0.1% by mass or more, more preferably 0.2% by mass or more, further more preferably 0.3% by mass or more, even more preferably 0.5% by mass or more, and preferably 5% by mass or less, more preferably 3% by mass or less, further more preferably 2% by mass or less, even more preferably 1% by mass or less, in the total composition from the viewpoint of improving emulsifiability, stability of the oil-in-water-type emulsion composition, an absorption feeling upon application to dry skin, a moist feeling after washing with water, easy spreading upon application to wet skin, the absence of sliminess upon application, a cover or protection feeling, next morning's moist feeling, a skin glow feeling after application to the skin and washing off with water, and the absence of stickiness. The content of the component (C) is preferably from 0.1 to 5% by mass, more preferably from 0.2 to 3% by mass, further more preferably from 0.3 to 2% by mass, even more preferably from 0.5 to 1% by mass, in the total composition.

When the component (C) is a cationic surfactant, a content thereof is preferably 0.1% by mass or more, more preferably 0.2% by mass or more, further more preferably 0.3% by mass or more, even more preferably 0.5% by mass or more, and preferably 5% by mass or less, more preferably 3% by mass or less, further more preferably 2% by mass or less, even more preferably 1% by mass or less, in the total composition from the viewpoint of improving emulsifiability, stability of the oil-in-water-type emulsion composition, an absorption feeling upon application to dry skin, a moist feeling after washing with water, easy spreading upon application to wet skin, the absence of sliminess upon application, a cover or protection feeling, next morning's moist feeling, a skin glow feeling after application to the skin and washing off with water, and the absence of stickiness. When the component (C) is a cationic surfactant, the content thereof is preferably from 0.1 to 5% by mass, more preferably from 0.2 to 3% by mass, further more preferably from 0.3 to 2% by mass, even more preferably from 0.5 to 1% by mass, in the total composition.

In the present invention, a mass ratio of the component (B) to the component (C), (B) / (C), is preferably 0.1 or more, more preferably 1 or more, further more preferably 2.8 or more, and preferably 20 or less, more preferably 6.8 or less, and further more preferably 5 or less, from the viewpoint of improving stability of the oil-in-water-type emulsion composition, an absorption feeling upon application to dry skin, a moist feeling after washing with water, easy spreading upon application to wet skin, the absence of sliminess upon application, a cover or protection feeling, next morning's moist feeling, a skin glow feeling after application to the skin and washing off with water, and the absence of stickiness. The mass ratio of the component (B) to the component (C), (B) / (C), is preferably from 0.1 to 20, more preferably from 1 to 6.8, further more preferably from 2.8 to 5.

The component (D) is one or more members selected from the group consisting of a ceramide, a hydrocarbon oil which is in a paste state at 25°C, and an ester oil which is in a paste state at 25°C.

In the present invention, the paste state refers to a state in which a viscosity at 25°C is larger than 10 000 mPa·s. The viscosity is measured in the same manner as in the component (A). The paste state also refers to a state in which a consistency is from 100 to 430. In this context, the consistency is measured in accordance with JIS K 2235 5.10. JIS K 2235 5.10 provides a measurement method for petroleum wax. In the present invention, the component (D) other than petroleum wax is also measured in accordance with JIS K 2235 5.10.

The hydrocarbon oil which is in a paste state at 25°C or the ester oil which is in a paste state at 25°C as the component (D) has a consistency of preferably 130 or more, more preferably 150 or more, further more preferably 170 or more, and preferably 400 or less, more preferably 350 or less, further more preferably 300 or less, even more preferably 250 or less. The consistency is preferably from 130 to 400, more preferably from 130 to 350, further more preferably from 150 to 300, even more preferably from 170 to 250.

Examples of the ceramide as the component (D) include natural ceramide and sphingosine derivatives as well as substances having a ceramide-like structure described in JP-A-S62-228048, JP-A-S63-216812, JP-A-S63-227513, JP-A-S64-29347, JP-A-S64-31752, and JP-A-H08-319263. Specifically, a compound of a formula selected from the group consisting of the following formulas (2) and (3) is preferred, and a compound of the formula (2) is more preferred, from the viewpoint of improving a moist feeling after application to dry skin and washing with water, a cover or protection feeling after application to wet skin, next morning's moist feeling, and a skin glow feeling after application to the skin and washing off with water:

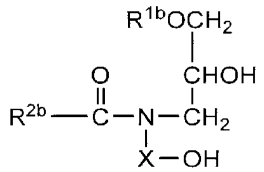
(2)

wherein $R^{1b}$ represents a hydrocarbon group having 10 to 26 carbon atoms, $R^{2b}$ represents a hydrocarbon group having 9 to 25 carbon atoms, and X represents $-(CH_2)_n-$(wherein n represents an integer of 2 to 6),

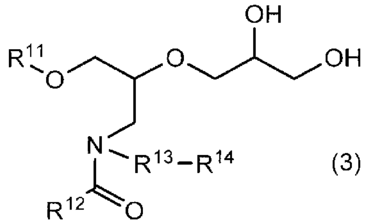
(3)

wherein $R^{11}$ and $R^{12}$ are the same or different and each represent an optionally hydroxylated hydrocarbon group having 1 to 40 carbon atoms, $R^{13}$ represents an alkylene group having 1 to 6 carbon atoms or a single bond, and $R^{14}$ represents a hydrogen atom, an alkoxy group having 1 to 12 carbon atoms, or a 2,3-dihydroxypropyloxy group, provided that when $R^{13}$ is a single bond, $R^{14}$ is a hydrogen atom.

In the formulas (2) and (3), the hydrocarbon group is preferably an alkyl group or an alkenyl group.

Examples of the compound of the formula (2) include N-(hexadesiloxyhydroxypropyl)-N-hydroxyethylhexadecanamide. Examples of the compound of the formula (3) include long-chain dibasic acid bis-3-methoxypropylamide.

The compound of the formula (2) is a pseudo-ceramide and is a functional ceramide component, which can make up for the work of ceramide and improve skin conditions (e.g., a moisture content).

Examples of the hydrocarbon oil in a paste state as the component (D) include petrolatum and hydrocarbon oils.

Examples of the ester oil in a paste state as the component (D) include: shea butter which is a plantderived wax; cholesterol derivatives such as cholesteryl isostearate, cholesteryl hydroxystearate, macadamia nut oil fatty acid cholesteryl, di(cholesteryl/behenyl/octyldodecyl) N-lauroyl-L-glutamate, and lanolin fatty acid cholesteryl;

phytosterol derivatives such as di(phytosteryl/behenyl/2-octyldodecyl) N-lauroyl-L-glutamate, phytosteryl isostearate, phytosteryl oleate, and di(phytosteryl/isostearyl/cetyl/stearyl/behenyl) dilinoleate;

dipentaerythritol fatty acid ester such as dipentaerythritol hexaoxystearate, dipentaerythritol rosinate, and dipentaerithrityl tri-polyhydroxystearate; triglycerides such as caprylic/capric/myristic/stearic triglyceride, trilanolin fatty acid glyceryl, and glyceryl tribehenate;

partially hydrogenated triglycerides such as hydrogenated oils; lanolin, soft lanolin fatty acid, lanosterol derivatives, and lanolin fatty acid ester;

dimer dilinoleic acid ester such as hydrogenated castor oil dimer dilinoleate, (phytosteryl/isostearyl/cetyl/stearyl/behenyl) dimer dilinoleate, bis(behenyl/isostearyl/phytosteryl) dimer dilinoleyl dimer dilinoleate, and bis(behenyl/isostearyl/phytosteryl) dimer dilinoleyl dimer dilinoleate, glyceryl hydrogenated rosinate; diglyceryl adipate mixed fatty acid ester such as bisdiglyceryl polyacyladipate-2; and

acylamino acid ester such as di(phytosteryl/behenyl/octyldodecyl) N-lauroyl-L-glutamate, di(phytosteryl/behenyl/octyldodecyl) N-lauroyl-L-glutamate, and di(phytosteryl/behenyl/2-octyldodecyl) N-lauroyl-L-glutamate.

The component (D) preferably comprises one or more members selected from the group consisting of dimer dilinoleic acid ester, acylamino acid ester, petrolatum, and a ceramide, more preferably comprises one or more members selected from the group consisting of petrolatum and a ceramide, further more preferably comprises a ceramide, and even more preferably comprises N-(hexadesiloxyhydroxypropyl)-N-hydroxyethylhexadecanamide as the ceramide, from the

viewpoint of improving stability of the oil-in-water-type emulsion composition, an absorption feeling upon application to dry skin, a moist feeling after washing with water, easy spreading upon application to wet skin, the absence of sliminess upon application, a cover or protection feeling, next morning's moist feeling, a skin glow feeling after application to the skin and washing off with water, and the absence of stickiness.

The component (D) preferably contains both of one or more members selected from the group consisting of a hydrocarbon oil which is in a paste state at 25°C and an ester oil which is in a paste state at 25°C, and one or more members selected from the group consisting of a ceramide, more preferably contains both of one or more members selected from the group consisting of a hydrocarbon oil which is in a paste state at 25°C and one or more members selected from the group consisting of a ceramide, further more preferably contains both of petrolatum and one or more members selected from the group consisting of a ceramide, and even more preferably contains both of petrolatum and N-(hexadesiloxyhydroxypropyl)-N-hydroxyethylhexadecanamide, from the viewpoint of improving stability of the oil-in-water-type emulsion composition, an absorption feeling upon application to dry skin, a moist feeling after washing with water, easy spreading upon application to wet skin, the absence of sliminess upon application, a cover or protection feeling, next morning's moist feeling, a skin glow feeling after application to the skin and washing off with water, and the absence of stickiness.

A ceramide is contained as the component (D) at preferably 0.01% by mass or more, more preferably 0.05% by mass or more, further more preferably 1% by mass or more, even more preferably 2% by mass or more, and preferably 20% by mass or less, more preferably 10% by mass or less, further more preferably 8% by mass or less, even more preferably 6% by mass or less, particularly preferably 4% by mass or less, in the total composition from the viewpoint of improving a moist feeling after application to dry skin and washing with water, a cover or protection feeling after application to wet skin, next morning's moist feeling, and a skin glow feeling after application to the skin and washing off with water. A ceramide is contained as the component (D) at preferably from 0.01 to 20% by mass, more preferably from 0.01 to 10% by mass, further more preferably from 0.05 to 8% by mass, even more preferably from 1 to 6% by mass, particularly preferably from 2 to 4% by mass, in the total composition.

One or more components (D) can be used. A content thereof is preferably 0.01% by mass or more, more preferably 0.1% by mass or more, further more preferably 0.5% by mass or more, even more preferably 1% by mass or more, especially preferably 2% by mass or more, particularly preferably 3% by mass or more, and preferably 30% by mass or less, more preferably 20% by mass or less, further more preferably 15% by mass or less, even more preferably 12% by mass or less, especially preferably 10% by mass or less, particularly preferably 7% by mass or less, in the total composition from the viewpoint of improving stability of the oil-in-water-type emulsion composition, an absorption feeling upon application to dry skin, a moist feeling after washing with water, easy spreading upon application to wet skin, the absence of sliminess upon application, a cover or protection feeling, next morning's moist feeling, a skin glow feeling after application to the skin and washing off with water, and the absence of stickiness. The content of the component (D) is preferably from 0.01 to 30% by mass, more preferably from 0.1 to 20% by mass, further more preferably from 0.5 to 15% by mass, even more preferably from 1 to 12% by mass, especially preferably from 2 to 10% by mass, particularly preferably from 3 to 7% by mass, in the total composition.

In the present invention, a mass ratio of the component (C) to the component (D), (C) / (D), is preferably 0.05 or more, more preferably 0.1 or more, further more preferably 0.14 or more, and preferably 1 or less, more preferably 0.23 or less, further more preferably 0.18 or less, from the viewpoint of improving stability, an absorption feeling upon application to dry skin, a moist feeling after washing with water, easy spreading upon application to wet skin, the absence of sliminess upon application, a cover or protection feeling, next morning's moist feeling, a skin glow feeling after application to the skin and washing off with water, and the absence of stickiness. The mass ratio of the component (C) to the component (D), (C) / (D), is preferably from 0.05 to 1, more preferably from 0.1 to 0.23, further more preferably from 0.14 to 0.18.

In the present invention, a content of water as the component (E) is preferably 30% by mass or more, more preferably 35% by mass or more, further more preferably 40% by mass or more, and preferably 90% by mass or less, more preferably 70% by mass or less, further more preferably 60% by mass or less, in the total composition from the viewpoint of improving stability of the oil-in-water-type emulsion composition, an absorption feeling upon application to dry skin, easy spreading upon application to wet skin, and the absence of sliminess upon application. The content of water as the component (E) is preferably from 30 to 90% by mass, more preferably from 35 to 70% by mass, further more preferably from 40 to 60% by mass, in the total composition.

The oil-in-water-type emulsion composition of the present invention can further contain (F) a nonionic surfactant having HLB of 13 or more.

In this context, HLB (hydrophilic-lipophilic balance) refers to the molecular weight of a hydrophilic group moiety based on the total molecular weight of the surfactant, and is determined according to the Griffin equation as to nonionic surfactants.

HLB of a mixed surfactant constituted by two or more nonionic surfactants is an arithmetic average of the respective HLB values of the nonionic surfactants based on the mixing ratio.

$$\text{Mixed HLB} = \Sigma(\text{HLBx} \times \text{Wx}) / \Sigma\text{Wx}.$$

HLBx represents the HLB value of nonionic surfactant X.

Wx represents the weight (g) of the nonionic surfactant X having the value of HLBx.

The nonionic surfactant may not be limited as long as it can be used in usual cosmetics. Examples thereof include polyoxyethylene fatty acid ester, polyoxyethylene sorbitan fatty acid ester, polyoxyethylene alkyl ether, polyoxyethylene sorbitol fatty acid ester, polyoxyethylene glycerin fatty acid ester, polyoxyethylene propylene glycol fatty acid ester, polyoxyethylene castor oil, polyoxyethylene hydrogenated castor oil, polyoxyethylene hydrogenated castor oil fatty acid ester, polyoxyethylene phytostanol ether, polyoxyethylene phytosterol ether, polyoxyethylene cholestanol ether, polyoxyethylene cholesteryl ether, silicone-based surfactants such as polyether-modified silicone, sucrose fatty acid ester, glycerin fatty acid ester, alkyl saccharide, and alkyl glyceryl ether.

Among them, the nonionic surfactant preferably comprises one or more members selected from the group consisting of polyoxyethylene sorbitan fatty acid ester and polyoxyethylene hydrogenated castor oil and more preferably comprises polyoxyethylene sorbitan fatty acid ester, from the viewpoint of preventing oil components from being unlikely to remain on the skin when toweled or washed off with water after application to wet skin, and improving stability of the oil-in-water-type emulsion composition, an absorption feeling upon application to dry skin, easy spreading upon application to wet skin, and the absence of sliminess upon application.

One or more nonionic surfactants can be used as the component (F). A content thereof is preferably less than 0.1% by mass, more preferably less than 0.05% by mass, in the total composition, and further more preferably, substantially no such nonionic surfactant is contained, from the viewpoint of preventing oil components from being unlikely to remain on the skin when toweled or washed off with water after application to wet skin, and improving an absorption feeling upon application to dry skin, a moist feeling after washing with water, a cover or protection feeling upon application to wet skin, next morning's moist feeling, and a skin glow feeling after application to the skin and washing off with water.

The oil-in-water-type emulsion composition of the present invention may further contain a nonionic surfactant having HLB of less than 13.

The nonionic surfactant having HLB of less than 13 may not be limited as long as it can be used in usual cosmetics. Examples thereof include polyoxyethylene fatty acid ester, polyoxyethylene sorbitan fatty acid ester, polyoxyethylene alkyl ether, polyoxyethylene sorbitol fatty acid ester, polyoxyethylene glycerin fatty acid ester, polyoxyethylene propylene glycol fatty acid ester, polyoxyethylene castor oil, polyoxyethylene hydrogenated castor oil, polyoxyethylene hydrogenated castor oil fatty acid ester, polyoxyethylene phytostanol ether, polyoxyethylene phytosterol ether, polyoxyethylene cholestanol ether, polyoxyethylene cholesteryl ether, silicone-based surfactants such as polyether-modified silicone, sucrose fatty acid ester, glycerin fatty acid ester, alkyl saccharide, and alkyl glyceryl ether.

The nonionic surfactant having HLB of less than 13 preferably has HLB of less than 12, more preferably less than 10, further more preferably less than 8, from the viewpoint of improving a cover or protection feeling after application to wet skin, next morning's moist feeling, and a skin glow feeling after application to the skin and washing off with water.

Among them, the nonionic surfactant preferably comprises one or more members selected from the group consisting of polyether-modified silicone, glycerin fatty acid ester, and sorbitan fatty acid ester and more preferably comprises glycerin fatty acid ester, from the viewpoint of preventing oil components from being unlikely to remain on the skin when toweled or washed off with water after application to wet skin, and improving stability of the oil-in-water-type emulsion composition, an absorption feeling upon application to dry skin, easy spreading upon application to wet skin, and the absence of sliminess upon application.

One or more nonionic surfactants having HLB of less than 13 can be used. A content thereof is preferably 3% by mass or less, more preferably 2% by mass or less, further more preferably 1% by mass or less, even more preferably 0.5% by mass or less, in the total composition, and particularly preferably, substantially no such nonionic surfactant is contained, from the viewpoint of preventing oil components from being unlikely to remain on the skin when toweled or washed off with water after application to wet skin, and improving an absorption feeling upon application to dry skin, a moist feeling after washing with water, a cover or protection feeling upon application to wet skin, next morning's moist feeling, and a skin glow feeling after application to the skin and washing off with water.

The oil-in-water-type emulsion composition of the present invention can further contain (G) a cationic polymer, which allows the oil-in-water-type emulsion composition to blend well with the skin so as to improve an absorption feeling to the skin, facilitates oil components remaining on the skin when toweled or washed off with water after application to wet skin, and can improve an absorption feeling upon application to dry skin, a moist feeling after washing with water, a cover or protection feeling upon application to wet skin, next morning's moist feeling, and a skin glow feeling after application to the skin and washing off with water.

Examples of the cationic polymer include cationized cellulose, cationized starch, cationized guar gum, cationized tara gum, cationized locust bean gum, cationized fenugreek gum, cationized xanthan gum, polymers of diallyl dialkyl quaternary ammonium salts, diallyl dialkyl quaternary ammonium salt/acrylamide copolymers, diallyl dialkyl quaternary

ammonium salt/acrylamide/acrylic acid copolymers, vinylimidazolium trichloride/vinylpyrrolidone copolymers, hydroxyethylcellulose/dimethyldiallylammonium chloride copolymers, vinylpyrrolidone/quaternized dimethylaminoethyl methacrylate copolymers, polyvinylpyrrolidone/alkylamino acrylate copolymers, polyvinylpyrrolidone/alkylamino acrylate/vinylcaprolactam copolymers, vinylpyrrolidone/methacrylamidopropyl trimethylammonium chloride copolymers, alkylacrylamide/acrylate/alkylaminoalkylacrylamide/polyet hylene glycol methacrylate copolymers, polymethacryloylethyldimethyl betaine, ethyl[(methacryloyloxy)ethyl]dimethylammonium ethyl sulfate/N,N-dimethylacrylamide/polyethylene glycol dimethacrylate copolymers, poly(methacryloyloxyethyl trimethylammonium chloride), quaternized dimethylaminoethyl methacrylate/dialkylacrylamide/polyethylene glycol dimethacrylate copolymers, adipic acid/dimethylaminohydroxypropylethylene triamine copolymers (Sandoz Inc., USA, Cartaletin), and cationic polymers described in JP-A-S53-139734 and JP-A-S60-36407.

Among them, the cationic polymer preferably comprises one or more members selected from the group consisting of a vinylpyrrolidone/quaternized dimethylaminoethyl methacrylate copolymer, a diallyl dialkyl quaternary ammonium salt/acrylamide copolymer, a diallyl dialkyl quaternary ammonium salt/acrylamide/acrylic acid copolymer, a quaternized dimethylaminoethyl methacrylate/dialkylacrylamide/polyethylene glycol dimethacrylate copolymer, cationized guar gum, cationized cellulose, an ethyl[(methacryloyloxy)ethyl]dimethylammonium ethyl sulfate/N,N-dimethylacrylamide/polyethylene glycol dimethacrylate copolymer, and poly(methacryloyloxyethyl trimethylammonium chloride), more preferably comprises one or more members selected from the group consisting of a diallyl dialkyl quaternary ammonium salt/acrylamide copolymer, a quaternized dimethylaminoethyl methacrylate/dialkylacrylamide/polyethylene glycol dimethacrylate copolymer, an ethyl[(methacryloyloxy)ethyl]dimethylammonium ethyl sulfate/N,N-dimethylacrylamide/polyethylene glycol dimethacrylate copolymer, and poly(methacryloyloxyethyl trimethylammonium chloride), further more preferably comprises one or more members selected from the group consisting of an ethyl[(methacryloyloxy)ethyl] dimethylammonium ethyl sulfate/N,N-dimethylacrylamide/polyethylene glycol dimethacrylate copolymer and poly(methacryloyloxyethyl trimethylammonium chloride), and even more preferably comprises an ethyl[(methacryloyloxy)ethyl]dimethylammonium ethyl sulfate/N,N-dimethylacrylamide/polyethylene glycol dimethacrylate copolymer, from the viewpoint of facilitating oil components remaining on the skin when toweled or washed off with water after application to wet skin, and improving an absorption feeling upon application to dry skin, a moist feeling after washing with water, a cover or protection feeling upon application to wet skin, next morning's moist feeling, and a skin glow feeling after application to the skin and washing off with water.

One or more components (G) can be used. A larger content thereof is preferred from the viewpoint of facilitating oil components remaining on the skin when toweled or washed off with water after application to wet skin, and improving a moist feeling after application to dry skin and washing with water, a cover or protection feeling upon application to wet skin, next morning's moist feeling, and a skin glow feeling after application to the skin and washing off with water. On the other hand, a smaller content thereof is preferred from the viewpoint of improving an absorption feeling upon application to dry skin, the absence of sliminess upon application to the skin wetted with water, and the absence of stickiness after application to wet skin and rinsing with water. Accordingly, the content thereof is preferably 0.001% by mass or more, more preferably 0.01% by mass or more, further more preferably 0.02% by mass or more, and preferably 1% by mass or less, more preferably 0.5% by mass or less, further more preferably 0.2% by mass or less, even more preferably 0.15% by mass or less, in the total composition from the viewpoint of improving a moist feeling after application to dry skin and washing with water, a cover or protection feeling upon application to wet skin, next morning's moist feeling, and a skin glow feeling after application to the skin and washing off with water, and from the viewpoint of improving an absorption feeling upon application to dry skin, the absence of sliminess upon application to the skin wetted with water, and the absence of stickiness after application to wet skin and rinsing with water. The content of the component (G) is preferably from 0.001 to 1% by mass, more preferably from 0.01 to 0.5% by mass, further more preferably from 0.02 to 0.2% by mass, even more preferably from 0.02 to 0.15% by mass, in the total composition.

The oil-in-water-type emulsion composition of the present invention can further contain (H) tocopherol and/or a derivative thereof, which cooperates with oil components including the components (A), (B), and (D) so as to improve the affinity of the oil components in the oil-in-water-type emulsion composition for the skin and improve absorption or sorption properties to the skin, and therefore facilitates the oil components remaining on the skin when toweled or washed off with water after application to wet skin, and can improve an absorption feeling upon application to dry skin, a moist feeling after washing with water, a cover or protection feeling upon application to wet skin, next morning's moist feeling, and a skin glow feeling after application to the skin and washing off with water.

In this context, the tocopherol is a water-insoluble component and includes natural vitamin E (i.e., a mixture of d-α, d-β, d-γ, and d-δ-tocopherols), β-, γ-, or δ-tocopherol, and a mixture thereof. Examples of the tocopherol derivative include tocopherol precursors, particularly, tocopherol ester, for example, tocopherol acetate, tocopherol nicotinate, tocopherol succinate, tocopherol linoleate, and tocopherol oleate.

Among them, a component with an unmodified hydroxy group in the molecular skeleton of tocopherol is more preferable than an ester form with a modified hydroxy group in the molecular skeleton of tocopherol, from the viewpoint of cooperating with oil components including the components (A), (B), and (D) so as to improve the affinity of the oil

components in the oil-in-water-type emulsion composition for the skin and improve an absorption feeling to the skin, and therefore facilitating the oil components remaining on the skin when toweled or washed off with water after application to wet skin, and improving an absorption feeling upon application to dry skin, a moist feeling after washing with water, a cover or protection feeling upon application to wet skin, next morning's moist feeling, and a skin glow feeling after application to the skin and washing off with water. The component (H) preferably comprises one or more members selected from the group consisting of natural vitamin E and d-δ-tocopherol from the viewpoint of cooperating with oil components including the components (A), (B), and (D) so as to improve the affinity of the oil components in the oil-in-water-type emulsion composition for the skin and improve an absorption feeling to the skin, and therefore facilitating the oil components remaining on the skin when toweled or washed off with water after application to wet skin, and improving an absorption feeling upon application to dry skin, a moist feeling after washing with water, a cover or protection feeling upon application to wet skin, next morning's moist feeling, and a skin glow feeling after application to the skin and washing off with water.

One or more components (H) can be used. A content thereof is preferably 0.01% by mass or more, more preferably 0.04% by mass or more, further more preferably 0.05% by mass or more, even more preferably 0.08% by mass or more, and preferably 1% by mass or less, more preferably 0.5% by mass or less, further more preferably 0.3% by mass or less, even more preferably 0.2% by mass or less, in the total composition from the viewpoint of cooperating with oil components including the components (A), (B), and (D) so as to improve the affinity of the oil components in the oil-in-water-type emulsion composition for the skin and improve an absorption feeling to the skin, and therefore facilitating the oil components remaining on the skin when toweled or washed off with water after application to wet skin, and improving an absorption feeling upon application to dry skin, a moist feeling after washing with water, a cover or protection feeling upon application to wet skin, next morning's moist feeling, and a skin glow feeling after application to the skin and washing off with water, and from the viewpoint of preventing too large a content from impairing stability of the oil-in-water-type emulsion composition, an absorption feeling after application to dry skin, easy spreading upon application to wet skin, and the absence of stickiness after application to wet skin and washing off with water. The content of the component (H) is preferably from 0.01 to 1% by mass, more preferably from 0.04 to 0.5% by mass, further more preferably from 0.05 to 0.3% by mass, even more preferably from 0.08 to 0.2% by mass, in the total composition.

In the present invention, the oil-in-water-type emulsion composition preferably contains the components (G) and (H) from the viewpoint of improving absorption or sorption properties of the oil-in-water-type emulsion composition to the skin, and therefore facilitating oil components remaining on the skin when toweled or washed off with water after application to wet skin, and improving an absorption feeling upon application to dry skin, a moist feeling after washing with water, a cover or protection feeling upon application to wet skin, next morning's moist feeling, and a skin glow feeling after application to the skin and washing off with water.

The oil-in-water-type emulsion composition of the present invention can contain, in addition to the components described above, components usually used in cosmetics, for example, an oil component other than the components (A), (B), and (D), a surfactant other than the components (C) and (F), a water-soluble polymer other than the component (G), an antioxidant, a fragrance, an antiseptic, a pH adjuster, a blood circulation promoter, a cooling agent, an antiperspirant, a germicide, a skin activator, a humectant, a freshener, and a colorant.

However, a content of an anionic surfactant is preferably 0.5% by mass or less, more preferably 0.1% by mass or less, in the total composition, and further more preferably, substantially no anionic surfactant is contained, for keeping the stability of the composition, allowing the oil-in-water-type emulsion composition to blend well with the skin so as to improve absorption or sorption properties to the skin, facilitating oil components remaining on the skin when toweled or washed off with water after application to wet skin, and improving an absorption feeling upon application to dry skin, a moist feeling after washing with water, a cover or protection feeling upon application to wet skin, next morning's moist feeling, and a skin glow feeling after application to the skin and washing off with water.

The oil-in-water-type emulsion composition of the present invention can be produced in accordance with a usual method.

The oil-in-water-type emulsion composition of the present invention is suitable as an oil-in-water-type emulsion cosmetic and can be applied to skin cosmetics including: makeup cosmetics such as makeup bases, foundations, concealers, blushes, eye shadows, mascara, eyeliners, eyebrow cosmetics, overcoats, and lipsticks; sun protection cosmetics such as sunscreen emulsions and sunscreen creams; and skin care cosmetics such as skin care emulsions, skin care creams, BB creams, and beauty essences. Among them, the oil-in-water-type emulsion composition is preferably a skin care cosmetic, more preferably a skin care emulsion or a skin care cream, further more preferably a skin care cream.

The oil-in-water-type emulsion composition of the present invention is absorbed to the skin when applied to dry skin, and therefore maintains an absorption feeling or a moist feeling even after being subsequently washed off with water. The oil-in-water-type emulsion composition, when applied to the skin wetted with water, needs no time for toweling the wet skin in advance, and is easy to spread on the wet skin and can be applied without taking time. In addition, the oil-in-water-type emulsion composition is applied without sliminess, and remains on the skin when toweled and therefore produces a cover or protection feeling. The oil-in-water-type emulsion composition produces a skin glow feeling and produces

favorable use impression without a sticky feeling, because the oil components of the oil-in-water-type emulsion composition remain, when applied to the skin wetted with water and washed off with water. Moreover, the oil-in-water-type emulsion composition is also excellent in stability.

The oil-in-water-type emulsion composition of the present invention can be used by application to the skin wetted with water. Also, the oil-in-water-type emulsion composition can be used by application to the skin followed by rinsing with water.

The oil-in-water-type emulsion composition of the present invention can be used by application to dry skin and additionally, can be used by application to the skin wetted with water. Furthermore, the oil-in-water-type emulsion composition may be used by application to the skin wetted with water and rising with a shower or the like.

The oil-in-water-type emulsion composition of the present invention is easy to spread over the skin wetted with water throughout the body after bathing, and can remain on the skin when toweled.

The oil components in the oil-in-water-type emulsion composition of the present invention can remain on the skin even washed off with water after application.

Since the oil components in the oil-in-water-type emulsion composition of the present invention remain on the skin in any case, particularly, an effect of a moisturizing component can be sufficiently obtained.

The oil-in-water-type emulsion composition of the present invention, particularly when applied to wet skin, (1) takes no time for skin care because the oil-in-water-type emulsion composition eliminates the need of toweling the wet skin in advance and in addition, is easy to spread over the skin and can be easily applied, (2) is applied without sliminess, and (3), in the case of bathing, can prevent the skin from starting to dry because the oil-in-water-type emulsion composition can be applied directly to wet skin immediately after bathing, though the skin which has lost a moisturizing component in the stratum corneum (stratum corneum intercellular lipid such as ceramide, and NMF) by bathing starts to dry immediately after bathing. Hence, application to wet skin is more suitable as a use method than application to dry skin. On the other hand, the oil-in-water-type emulsion composition may be applied to wet skin and washed off with water. A use method involving application to wet skin without washing off with water is more preferable than a use method involving washing off with water because non-oil components in the oil-in-water-type emulsion composition can also remain on the skin.

In relation to the embodiments mentioned above, the present invention further discloses the following compositions and the like.

<1> An oil-in-water-type emulsion composition comprising the following components (A), (B), (C), (D), and (E):

(A) a hydrocarbon oil which is in a liquid state at 25°C,
(B) one or more members selected from the group consisting of a higher fatty acid, a higher alcohol, and an ester oil which are in a liquid state at 25°C,
(C) from 0.1 to 5% by mass of one or more members selected from the group consisting of a cationic surfactant and an amphoteric surfactant,
(D) from 2 to 20% by mass of one or more members selected from the group consisting of a ceramide, a hydrocarbon oil which is in a paste state at 25°C, and an ester oil which is in a paste state at 25°C, and
(E) water, wherein

a total content of the components (A) and (B), (A) + (B), is from 1 to 30% by mass, and
a mass ratio of the component (B) to the total amount of the components (A) and (B), (B) / ((A) + (B)), is from 0.05 to 0.9.

<2> The oil-in-water-type emulsion composition according to <1>, wherein the component (A) preferably comprises one or more members selected from the group consisting of squalane, liquid paraffin, liquid isoparaffin, and an $\alpha$-olefin oligomer, more preferably comprises one or more members selected from the group consisting of liquid paraffin, liquid isoparaffin, and an $\alpha$-olefin oligomer, and further more preferably comprises liquid paraffin.

<3> The oil-in-water-type emulsion composition according to <1> or <2>, wherein a content of the component (A) is preferably 0.1% by mass or more, more preferably 1% by mass or more, further more preferably 2% by mass or more, even more preferably 4% by mass or more, and preferably 20% by mass or less, more preferably 10% by mass or less, further more preferably 7% by mass or less, even more preferably 6.5% by mass or less, particularly preferably 6% by mass or less, in the total composition.

<4> The oil-in-water-type emulsion composition according to any one of <1> to <3>, wherein the component (B) preferably comprises a higher fatty acid and more preferably comprises one or more members selected from the group consisting of oleic acid and isostearic acid.

<5> The oil-in-water-type emulsion composition according to any one of <1> to <4>, wherein the component (B) preferably comprises a higher alcohol and more preferably comprises one or more members selected from the group

consisting of isostearyl alcohol and oleyl alcohol.

<6> The oil-in-water-type emulsion composition according to any one of <1> to <5>, wherein the component (B) preferably comprises an ester oil, more preferably comprises one or more members selected from the group consisting of monoester oil, diester oil, and triester oil, further more preferably comprises diester oil, and even more preferably comprises acylamino acid diester.

<7> The oil-in-water-type emulsion composition according to any one of <1> to <6>, wherein the component (B) preferably comprises acylamino acid diester.

<8> The oil-in-water-type emulsion composition according to any one of <1> to <7>, wherein the component (B) preferably comprises one or more members selected from the group consisting of di(phytosteryl/2-octyldodecyl) **N-**lauroyl-L-glutamate, neopentyl glycol dicaprate, isononyl isononanoate, and caprylic/capric triglyceride and more preferably comprises di(phytosteryl/2-octyldodecyl) **N-**lauroyl-L-glutamate.

<9> The oil-in-water-type emulsion composition according to any one of <1> to <8>, wherein a content of the component (B) is preferably 0.5% by mass or more, more preferably 1% by mass or more, further more preferably 1.5% by mass or more, even more preferably 2% by mass or more, and preferably 10% by mass or less, more preferably 8% by mass or less, further more preferably 6% by mass or less, even more preferably 4% by mass or less, in the total composition.

<10> The oil-in-water-type emulsion composition according to any one of <1> to <9>, wherein a mass ratio of the component (A) to the component (B), (A) / (B), is preferably 0.1 or more, more preferably 0.45 or more, further more preferably 1.1 or more, and preferably 10 or less, more preferably 7 or less, further more preferably 3 or less.

<11> The oil-in-water-type emulsion composition according to any one of <1> to <10>, wherein a total content of the components (A) and (B), (A) + (B), is preferably 1% by mass or more, more preferably 2% by mass or more, further more preferably 3% by mass or more, even more preferably 5% by mass or more, and preferably 30% by mass or less, more preferably 20% by mass or less, further more preferably 15% by mass or less, even more preferably 10% by mass or less, in the total composition.

<12> The oil-in-water-type emulsion composition according to any one of <1> to <11>, wherein a mass ratio of the component (B) to the total amount of the components (A) and (B), (B) / ((A) + (B)), is preferably 0.1 or more, more preferably 0.15 or more, further more preferably 0.3 or more, even more preferably 0.35 or more, and preferably 0.8 or less, more preferably 0.75 or less, further more preferably 0.7 or less, even more preferably 0.5 or less.

<13> The oil-in-water-type emulsion composition according to any one of <1> to <12>, wherein the component (C) is a cationic surfactant, preferably a quaternary ammonium salt-type cationic surfactant, more preferably a quaternary ammonium salt-type cationic surfactant of the following formula (1):

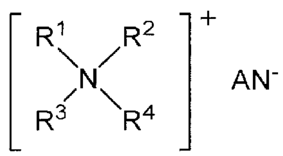
(1)

wherein

$R^1$ represents a linear, branched, or cyclic saturated or unsaturated hydrocarbon group having 6 or more carbon atoms,
$R^2$ represents an alkyl group having 1 to 4 carbon atoms,
$R^3$ and $R^4$ each independently represent an alkyl group having 1 to 4 carbon atoms or a linear, branched, or cyclic saturated or unsaturated hydrocarbon group having 6 or more carbon atoms, and
$AN^-$ represents a salt-forming anion.

<14> The oil-in-water-type emulsion composition according to any one of <1> to <12>, wherein the component (C) is an amphoteric surfactant, preferably comprises one or more members selected from the group consisting of an amino acid-based amphoteric surfactant, a sulfobetaine-type surfactant, an amidobetaine-type surfactant, and an imidazolinium betaine-type surfactant, more preferably comprises one or more members selected from the group consisting of an amino acid-based amphoteric surfactant, a sulfobetaine-type surfactant, and an amidobetaine-type surfactant, further more preferably comprises one or more members selected from the group consisting of an amino acid-based amphoteric surfactant and a sulfobetaine-type surfactant, and even more preferably comprises an amino acid-based surfactant.

<15> The oil-in-water-type emulsion composition according to any one of <1> to <13>, wherein the component (C) is preferably a cationic surfactant.

<16> The oil-in-water-type emulsion composition according to any one of <1> to <15>, wherein a content of the

component (C) is preferably 0.1% by mass or more, more preferably 0.2% by mass or more, further more preferably 0.3% by mass or more, even more preferably 0.5% by mass or more, and preferably 5% by mass or less, more preferably 3% by mass or less, further more preferably 2% by mass or less, even more preferably 1% by mass or less, in the total composition.

<17> The oil-in-water-type emulsion composition according to any one of <1> to <13> and <15>, wherein the component (C) is a cationic surfactant, and a content thereof is preferably 0.1% by mass or more, more preferably 0.2% by mass or more, further more preferably 0.3% by mass or more, even more preferably 0.5% by mass or more, and preferably 5% by mass or less, more preferably 3% by mass or less, further more preferably 2% by mass or less, even more preferably 1% by mass or less, in the total composition.

<18> The oil-in-water-type emulsion composition according to any one of <1> to <17>, wherein a mass ratio of the component (B) to the component (C), (B) / (C), is preferably 0.1 or more, more preferably 1 or more, further more preferably 2.8 or more, and preferably 20 or less, more preferably 6.8 or less, further more preferably 5 or less.

<19> The oil-in-water-type emulsion composition according to any one of <1> to <18>, wherein the component (D) is a hydrocarbon oil which is in a paste state at 25°C or an ester oil which is in a paste state at 25°C, and a consistency thereof is preferably 130 or more, more preferably 150 or more, further more preferably 170 or more, and preferably 400 or less, more preferably 350 or less, further more preferably 300 or less, even more preferably 250 or less.

<20> The oil-in-water-type emulsion composition according to any one of <1> to <18>, wherein the component (D) is a ceramide, preferably a compound of a formula selected from the group consisting of the following formulas (2) and (3), more preferably a compound of the formula (2), further more preferably N-(hexadesiloxyhydroxypropyl)-N-hydroxyethylhexadecanamide:

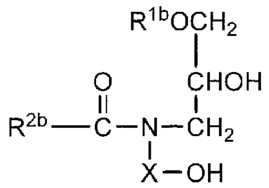

(2)

wherein $R^{1b}$ represents a hydrocarbon group having 10 to 26 carbon atoms, $R^{2b}$ represents a hydrocarbon group having 9 to 25 carbon atoms, and X represents $-(CH_2)_n-$(wherein n represents an integer of 2 to 6),

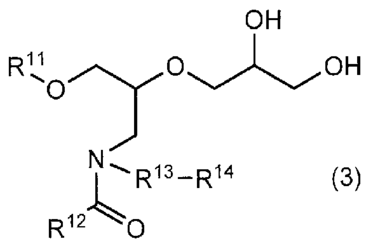

(3)

wherein $R^{11}$ and $R^{12}$ are the same or different and each represent an optionally hydroxylated hydrocarbon group having 1 to 40 carbon atoms, $R^{13}$ represents an alkylene group having 1 to 6 carbon atoms or a single bond, $R^{14}$ represents a hydrogen atom, an alkoxy group having 1 to 12 carbon atoms, or a 2,3-dihydroxypropyloxy group, provided that when $R^{13}$ is a single bond, $R^{14}$ is a hydrogen atom.

<21> The oil-in-water-type emulsion composition according to any one of <1> to <19>, wherein the component (D) is a hydrocarbon oil in a paste state, preferably petrolatum or a hydrocarbon oil.

<22> The oil-in-water-type emulsion composition according to any one of <1> to <19>, wherein the component (D) is an ester oil in a paste state, preferably shea butter, a cholesterol derivative, a phytosterol derivative, a dipentaerythritol fatty acid ester, a triglyceride, lanolin, soft lanolin fatty acid, a lanosterol derivative, lanolin fatty acid ester, dimer dilinoleic acid ester, diglyceryl adipate mixed fatty acid ester, or acylamino acid ester.

<23> The oil-in-water-type emulsion composition according to any one of <1> to <22>, wherein the component (D) preferably comprises one or more members selected from the group consisting of dimer dilinoleic acid ester, acylamino acid ester, petrolatum, and a ceramide, more preferably comprises one or more members selected from the group consisting of petrolatum and a ceramide, further more preferably comprises a ceramide, and even more preferably comprises N-(hexadesiloxyhydroxypropyl)-N-hydroxyethylhexadecanamide as the ceramide.

<24> The oil-in-water-type emulsion composition according to any one of <1> to <23>, wherein the component (D) preferably contains both of one or more members selected from the group consisting of a hydrocarbon oil which is in a paste state at 25°C and an ester oil which is in a paste state at 25°C, and one or more members selected from the group

consisting of a ceramide, more preferably contains both of one or more members selected from the group consisting of a hydrocarbon oil which is in a paste state at 25°C and one or more members selected from the group consisting of a ceramide, further more preferably contains both of petrolatum and one or more members selected from the group consisting of a ceramide, and even more preferably contains both of petrolatum and N-(hexadesiloxyhydroxypropyl)-N-hydroxyethylhexadecanamide.

<25> The oil-in-water-type emulsion composition according to any one of <1> to <24>, wherein a ceramide is contained as the component (D) at preferably 0.01% by mass or more, more preferably 0.05% by mass or more, further more preferably 1% by mass or more, even more preferably 2% by mass or more, and preferably 20% by mass or less, more preferably 10% by mass or less, further more preferably 8% by mass or less, even more preferably 6% by mass or less, further more preferably 4% by mass or less, in the total composition.

<26> The oil-in-water-type emulsion composition according to any one of <1> to <25>, wherein a content of the component (D) is preferably 0.01% by mass or more, more preferably 0.1% by mass or more, further more preferably 0.5% by mass or more, even more preferably 1% by mass or more, especially preferably 2% by mass or more, particularly preferably 3% by mass or more, and preferably 30% by mass or less, more preferably 20% by mass or less, further more preferably 15% by mass or less, even more preferably 12% by mass or less, especially preferably 10% by mass or less, particularly preferably 7% by mass or less, in the total composition.

<27> The oil-in-water-type emulsion composition according to any one of <1> to <26>, wherein a mass ratio of the component (C) to the component (D), (C) / (D), is preferably 0.05 or more, more preferably 0.1 or more, further more preferably 0.14 or more, and preferably 1 or less, more preferably 0.23 or less, further more preferably 0.18 or less.

<28> The oil-in-water-type emulsion composition according to any one of <1> to <27>, wherein a content of water as the component (E) is preferably from 30 to 90% by mass, more preferably from 35 to 70% by mass, further more preferably from 40 to 60% by mass, in the total composition.

<29> An oil-in-water-type emulsion composition comprising the following components (A), (B), (C), (D), and (E):

(A) a hydrocarbon oil which is in a liquid state at 25°C,
(B) one or more members selected from the group consisting of a higher fatty acid, a higher alcohol, and an ester oil which are in a liquid state at 25°C,
(C) from 0.1 to 5% by mass of a cationic surfactant,
(D) from 1 to 20% by mass of a ceramide, and
(E) water.

<30> The oil-in-water-type emulsion composition according to any one of <1> to <29>, wherein further a content of (F) a nonionic surfactant having HLB of 13 or more is preferably less than 0.1% by mass, more preferably less than 0.05% by mass, in the total composition, and further more preferably, substantially no such nonionic surfactant is contained.

<31> The oil-in-water-type emulsion composition according to <30>, wherein the component (F) preferably comprises one or more members selected from the group consisting of polyoxyethylene sorbitan fatty acid ester and polyoxyethylene hydrogenated castor oil and more preferably comprises polyoxyethylene sorbitan fatty acid ester.

<32> The oil-in-water-type emulsion composition according to any one of <1> to <31>, wherein further a content of a nonionic surfactant having HLB of less than 13 is preferably 3% by mass or less, more preferably 2% by mass or less, further more preferably 1% by mass or less, even more preferably 0.5% by mass or less, in the total composition, and particularly preferably, substantially no such nonionic surfactant is contained.

<33> The oil-in-water-type emulsion composition according to <32>, wherein the nonionic surfactant having HLB of less than 13 is preferably a nonionic surfactant having HLB of less than 12, more preferably a nonionic surfactant having HLB of less than 10, further more preferably a nonionic surfactant having HLB of less than 8.

<34> The oil-in-water-type emulsion composition according to <32> or <33>, wherein the nonionic surfactant having HLB of less than 13 preferably comprises one or more members selected from the group consisting of polyether-modified silicone, glycerin fatty acid ester, and sorbitan fatty acid ester and more preferably comprises glycerin fatty acid ester.

<35> The oil-in-water-type emulsion composition according to any one of <1> to <34>, preferably, further comprising (G) a cationic polymer.

<36> The oil-in-water-type emulsion composition according to <35>, wherein the component (G) preferably comprises one or more members selected from the group consisting of a vinylpyrrolidone/quaternized dimethylaminoethyl methacrylate copolymer, a diallyl dialkyl quaternary ammonium salt/acrylamide copolymer, a diallyl dialkyl quaternary ammonium salt/acrylamide/acrylic acid copolymer, a quaternized dimethylaminoethyl methacrylate/dialkylacrylamide/polyethylene glycol dimethacrylate copolymer, cationized guar gum, cationized cellulose, an ethyl[(methacryloyloxy)ethyl]dimethylammonium ethyl sulfate/N,N-dimethylacrylamide/polyethylene glycol dimethacrylate copolymer, and poly(methacryloyloxyethyl trimethylammonium chloride), more preferably comprises one or more members selected from the group consisting of a diallyl dialkyl quaternary ammonium salt/acrylamide copolymer, a quaternized

dimethylaminoethyl methacrylate/dialkylacrylamide/polyethylene glycol dimethacrylate copolymer, an ethyl[(methacryloyloxy)ethyl]dimethylammonium ethyl sulfate/N,N-dimethylacrylamide/polyethylene glycol dimethacrylate copolymer, and poly(methacryloyloxyethyl trimethylammonium chloride), further more preferably comprises one or more members selected from the group consisting of an ethyl[(methacryloyloxy)ethyl]dimethylammonium ethyl sulfate/N,N-dimethylacrylamide/polyethylene glycol dimethacrylate copolymer and poly(methacryloyloxyethyl trimethylammonium chloride), and even more preferably comprises an ethyl[(methacryloyloxy)ethyl]dimethylammonium ethyl sulfate/N,N-dimethylacrylamide/polyethylene glycol dimethacrylate copolymer.

<37> The oil-in-water-type emulsion composition according to <35> or <36>, wherein a content of the component (G) is preferably 0.001% by mass or more, more preferably 0.01% by mass or more, further more preferably 0.02% by mass or more, and preferably 1% by mass or less, more preferably 0.5% by mass or less, further more preferably 0.2% by mass or less, even more preferably 0.15% by mass or less, in the total composition.

<38> The oil-in-water-type emulsion composition according to any one of <1> to <37>, preferably, further comprising (H) tocopherol and a derivative thereof.

<39> The oil-in-water-type emulsion composition according to <38>, wherein the component (H) preferably comprises one or more members selected from the group consisting of natural vitamin E and d-δ-tocopherol.

<40> The oil-in-water-type emulsion composition according to <38> or <39>, wherein a content of the component (H) is preferably 0.01% by mass or more, more preferably 0.04% by mass or more, further more preferably 0.05% by mass or more, even more preferably 0.08% by mass or more, and preferably 1% by mass or less, more preferably 0.5% by mass or less, further more preferably 0.3% by mass or less, even more preferably 0.2% by mass or less, in the total composition.

<41> The oil-in-water-type emulsion composition according to any one of <35> to <40>, wherein the oil-in-water-type emulsion composition preferably contains the components (G) and (H).

<42> The oil-in-water-type emulsion composition according to any one of <1> to <41>, wherein a content of an anionic surfactant is preferably 0.5% by mass or less, more preferably 0.1% by mass or less, in the total composition, and further more preferably, substantially no anionic surfactant is contained.

<43> The oil-in-water-type emulsion composition according to any one of <1> to <42>, wherein the oil-in-water-type emulsion composition is preferably an oil-in-water-type emulsion cosmetic, more preferably a skin care cosmetic, further more preferably a skin care emulsion or a skin care cream, even more preferably a skin care cream.

<44> The oil-in-water-type emulsion composition according to any one of <1> to <43>, wherein the oil-in-water-type emulsion composition is used by application to the skin wetted with water.

<45> The oil-in-water-type emulsion composition according to any one of <1> to <43>, wherein the oil-in-water-type emulsion composition is used by application to the skin followed by rinsing with water.

<46> The oil-in-water-type emulsion composition according to any one of <1> to <43>, wherein the oil-in-water-type emulsion composition is used by application to wet skin without being washed off with water.

<47> An oil-in-water-type emulsion composition comprising the following components (A), (B), (C), (D), and (E):

(A) a hydrocarbon oil which is in a liquid state at 25°C,
(B) one or more members selected from the group consisting of a higher fatty acid, a higher alcohol, and an ester oil which are in a liquid state at 25°C and comprise acylamino acid ester,
(C) from 0.1 to 5% by mass of one or more members selected from the group consisting of a cationic surfactant and an amphoteric surfactant,
(D) one or more members selected from the group consisting of a ceramide, a hydrocarbon oil which is in a paste state at 25°C, and an ester oil which is in a paste state at 25°C, and
(E) water, wherein

a total content of the components (A) and (B), (A) + (B), is from 1 to 30% by mass, and
a mass ratio of the component (B) to the total amount of the components (A) and (B), (B) / ((A) + (B)), is from 0.05 to 0.9.

<48> An oil-in-water-type emulsion composition comprising the following components (A), (B), (C), (D), and (E):

(A) a hydrocarbon oil which is in a liquid state at 25°C,
(B) one or more members selected from the group consisting of a higher fatty acid, a higher alcohol, and an ester oil which are in a liquid state at 25°C and comprise acylamino acid ester,
(C) from 0.1 to 5% by mass of a cationic surfactant,
(D) a ceramide, and
(E) water.

<49> An oil-in-water-type emulsion composition to be subjected to a skin care method involving application to wet skin or a skin care method involving application to the skin followed by rinsing with water, the oil-in-water-type emulsion composition comprising the following components (A), (B), (C), (D), and (E):

(A) a hydrocarbon oil which is in a liquid state at 25°C,
(B) one or more members selected from the group consisting of a higher fatty acid, a higher alcohol, and an ester oil which are in a liquid state at 25°C,
(C) from 0.1 to 5% by mass of one or more members selected from the group consisting of a cationic surfactant and an amphoteric surfactant,
(D) from 2 to 20% by mass of one or more members selected from the group consisting of a ceramide, a hydrocarbon oil which is in a paste state at 25°C, and an ester oil which is in a paste state at 25°C, and
(E) water, wherein

a total content of the components (A) and (B), (A) + (B), is from 1 to 30% by mass, and
a mass ratio of the component (B) to the total amount of the components (A) and (B), (B) / ((A) + (B)), is from 0.05 to 0.9.

<50> An oil-in-water-type emulsion composition to be subjected to a skin care method involving application to wet skin or a skin care method involving application to the skin followed by rinsing with water, the oil-in-water-type emulsion composition comprising the following components (A), (B), (C), (D), and (E):

(A) a hydrocarbon oil which is in a liquid state at 25°C,
(B) one or more members selected from the group consisting of a higher fatty acid, a higher alcohol, and an ester oil which are in a liquid state at 25°C,
(C) from 0.1 to 5% by mass of a cationic surfactant,
(D) from 2 to 20% by mass of a ceramide, and
(E) water.

Examples

Examples 1 to 24 and Comparative Examples 1 to 7

Each oil-in-water-type emulsion composition was produced using the components shown in Tables 1 to 4, and evaluated for success or failure of preparation of an emulsion (cream), stability after 1 week at room temperature, an absorption feeling upon application to dry skin, a moist feeling after application to dry skin and then washing with water, easy spreading when the oil-in-water-type emulsion composition was applied to the skin wetted with water, the absence of sliminess upon application when the oil-in-water-type emulsion composition was applied to the skin wetted with water, a cover or protection feeling when the oil-in-water-type emulsion composition was applied to the skin wetted with water, next morning's moist feeling when the oil-in-water-type emulsion composition was applied to the skin wetted with water, a skin glow feeling when the oil-in-water-type emulsion composition was applied to the skin wetted with water and showered off, and the absence of stickiness when the oil-in-water-type emulsion composition was applied to the skin wetted with water and showered off. The results are also shown in Tables 1 to 4.

In the tables, the content of components contained as a mixture refers to a content in terms of the starting material (mixture).

(Production method)

Polyol (glycerin or the like) heated to 85°C was mixed with a small amount of water and a cationic surfactant or an amphoteric surfactant to obtain a surfactant phase. Next, oil components dissolved by mixing at 85°C were gradually added to the surfactant phase. Further, remaining water-soluble components were gradually added thereto, and phase emulsification was performed by stirring and mixing. The mixture thus thoroughly stirred was then cooled to obtain an oil-in-water-type emulsion composition.

(Evaluation method)

(1) Success or failure of preparation of emulsion (cream) :

80 mL of each oil-in-water-type emulsion composition was placed in a 110 mL transparent glass container (vial bottle #11, Tokyo Garasu Kikai Co., Ltd.), which was then hermetically sealed and left standing at room temperature (25°C) for 12 hours. The appearance of the composition thus left standing was visually observed and assessed according to the following criteria.

Good: The sample has a homogeneous single layer and is found to have no separation of aqueous components or oil components. It is an emulsion in a cream state.
Poor: Water components or oil components are separated, and no emulsion in a cream state is retained as a whole.

(2) Stability after 1 week at room temperature:
80 mL of each oil-in-water-type emulsion composition was placed in a 110 mL transparent glass container (vial bottle #11, Tokyo Garasu Kikai Co., Ltd.), which was then hermetically sealed and left standing at room temperature (25°C) for 1 week. The appearance of the composition thus left standing was visually observed and assessed according to the following criteria.

Good: The sample has a homogeneous single layer and is found to have no separation of aqueous components or oil components. It is an emulsion in a cream state.
Poor: Water components or oil components are separated, and no emulsion in a cream state is retained as a whole.

(3) Absorption feeling upon application to dry skin:
Five expert panelists washed the forearm of one arm with Curel Foam Body Wash (manufactured by Kao Corp.), which was then showered off with water and thoroughly toweled. The "absorption feeling" was evaluated according to the criteria given below when 0.5 g of each oil-in-water-type emulsion composition was put on the palm of the other hand (not wet) and applied to the forearm thus washed and dried using the palm. The results are shown in a total score from the five panelists.

5: Get a feeling that absorption properties to the skin are very good.
4: Get a feeling that absorption properties to the skin are good.
3: Get a feeling that absorption properties to the skin are slightly good.
2: Get a feeling that absorption properties to the skin are not much good.
1: Get a feeling that absorption properties to the skin are not good.

(4) Moist feeling after application to dry skin and then washing with water:
Five expert panelists washed the forearm of one arm with Curel Foam Body Wash (manufactured by Kao Corp.), which was then showered off with water and thoroughly toweled. 0.5 g of each oil-in-water-type emulsion composition was put on the palm of the other hand (not wet) and spread over the forearm thus washed and dried. Approximately 30 minutes later, the oil-in-water-type emulsion composition-applied forearm was showered at approximately 40°C, and extra moisture was toweled off by dab. The "moist feeling" after showering was evaluated according to the criteria given below by comparison with that before showering. The results are shown in a total score from the five panelists.

5: The moist feeling is not changed from that before showering.
4: The moist feeling is rarely changed from that before showering.
3: The moist feeling is slightly reduced from that before showering and however, still exists.
2: The moist feeling is reduced from that before showering and however, slightly remains.
1: There is no moist feeling after showering.

(5) Easy spreading upon application to skin wetted with water:
Five expert panelists washed the forearm of one arm with Curel Foam Body Wash (manufactured by Kao Corp.) at night, which was then showered off with water. 0.5 g of each oil-in-water-type emulsion composition was put on the palm of the other hand which was slightly wet, and spread over the forearm thus lightly wetted by showering. Approximately 1 minute later, extra moisture was toweled off by dab. The "easy spreading" was evaluated according to the criteria given below when the oil-in-water-type emulsion composition was applied to the lightly wetted skin using the palm. The results are shown in a total score from the five panelists.

5: Very easily spread.
4: Easily spread.

3: Slightly easily spread.
2: Not easily spread.
1: Difficult to spread.

(6) Absence of sliminess upon application when oil-in-water-type emulsion composition was applied to skin wetted with water:
The "absence of sliminess" was evaluated according to the criteria given below when the oil-in-water-type emulsion composition was applied to the lightly wetted skin using the palm in the same manner as in (5). The results are shown in a total score from the five panelists.

5: Not slimy.
4: Rarely slimy.
3: Not much slimy.
2: Slightly slimy.
1: Slimy.

(7) Cover or protection feeling when oil-in-water-type emulsion composition was applied to skin wetted with water:
Each oil-in-water-type emulsion composition was spread over the lightly wetted forearm in the same manner as in (5). Approximately 1 minute later, extra moisture was toweled off by dab. Approximately 2 minutes later, the "feeling of being covered and protected with a film" of the skin was evaluated by five expert panelists according to the criteria given below. The results are shown in a total score from the five panelists.

5: Get a lot of a feeling of being covered and protected with a film of the skin.
4: Get a feeling of being covered and protected with a film of the skin.
3: Get a slight feeling of being covered and protected with a film of the skin.
2: Get a little bit feeling of being covered and protected with a film of the skin.
1: Get no feeling of being covered and protected with a film of the skin.

(8) Next morning's moist feeling when oil-in-water-type emulsion composition was applied to skin wetted with water:
Each oil-in-water-type emulsion composition was spread over the lightly wetted forearm in the same manner as in (5). Approximately 1 minute later, extra moisture was toweled off by dab. On the next morning, the skin moist feeling was evaluated according to the criteria given below by comparison with that at a site without application. The results are shown in a total score from the five panelists.

5: Very moist as compared with the site without application.
4: Moist as compared with the site without application.
3: Slightly moist as compared with the site without application.
2: Moist a little bit as compared with the site without application.
1: Not moist as in the site without application.

(9) Skin glow feeling when oil-in-water-type emulsion composition was applied to skin wetted with water and showered off:
Five expert panelists washed the forearm of one arm with Curel Foam Body Wash (manufactured by Kao Corp.) at night, which was then showered off with water. 0.5 g of each oil-in-water-type emulsion composition was put on the palm of the other hand which was slightly wet, and spread over the forearm thus lightly wetted by showering. Approximately 30 seconds later, the forearm was showered with water at approximately 40°C, and extra moisture was toweled off by dab. Then, the glow feeling of the arm that had received the applied oil-in-water-type emulsion composition was evaluated according to the criteria given below. The results are shown in a total score from the five panelists.

5: Get a lot of a glow feeling.
4: Get a glow feeling.
3: Get a slight glow feeling.
2: Get few glow feelings.
1: Get no glow feeling.

(10) Absence of stickiness when oil-in-water-type emulsion composition was applied to skin wetted with water and showered off:

The absence of stickiness of the arm that had received the applied oil-in-water-type emulsion composition in the same manner as in (9) was evaluated according to the criteria given below after showering. The results are shown in a total score from the five panelists.

5: Feel no stickiness.
4: Rarely feel stickiness.
3: Not feel much stickiness.
2: Slightly feel stickiness.
1: Feel stickiness.

[Table 1]

| Component name (% by mass) | | Example 1 | Example 2 | Example 3 | Example 4 | Example 5 | Example 6 | Example 7 | Example 8 |
|---|---|---|---|---|---|---|---|---|---|
| (A) | Squalane *1 | | | | | | | | |
| | Liquid paraffin *2 | 5.0 | 5.0 | 5.0 | 5.0 | 3.0 | 6.5 | 5.0 | 2.0 |
| (B) | Di(phytosteryl/2-octyldodecyl) N-lauroyl-L-glutamate *3 | 3.0 | 3.0 | 3.0 | 3.0 | 5.0 | 1.5 | 3.0 | 3.0 |
| | Neopentyl glycol dicaprate *4 | | | | | | | | 3.0 |
| | Isononyl isononanoate *5 | | | | | | | | |
| | Caprylic/capric triglyceride *6 | | | | | | | | |
| (C) | Distearyl dimethylammonium chloride *7 | 0.8 | 0.8 | 0.8 | 0.8 | 0.8 | 0.8 | 0.8 | 0.8 |
| | Behentrimonium chloride *8 | | | | | | | | |
| (F) | Polyoxyethylene sorbitan monostearate *10 | | | | | | | | |
| | Glyceryl behenate *11 | | | | | | | | |
| | Sorbitan monostearate *12 | | | | | | | | |
| (D) | Petrolatum *13 | | | | | | | | |
| | N-(Hexadesiloxyhydroxypropyl)-N-hvdroxvethylhexadecanamide *14 | 2.0 | 2.0 | 2.0 | 2.0 | 2.0 | 2.0 | 2.0 | 2.0 |
| | Cholesteryl isostearate *15 | 3.0 | 3.0 | 3.0 | 3.0 | 3.0 | 3.0 | 3.0 | 3.0 |
| | Di(phytosteryl/behenyl/2-octyldodecyl) N-lauroyl-L-glutamate *16 | | | | | | | | |
| | Di(phytosteryl/isostearyl/cetyl/stearyl/behenyl) dilinoleate *17 | | | | | | | | |
| | Concentrated glycerin *18 | | | | | | | | |
| | Sorbitol *19 | 20.0 | 20.0 | 20.0 | 20.0 | 20.0 | 20.0 | 20.0 | 20.0 |
| | Polyethylene glycol 600 *20 | 8.0 | 8.0 | 8.0 | 8.0 | 8.0 | 8.0 | 8.0 | 8.0 |
| (H) | d-δ-Tocopherol *21 | 3.0 | 3.0 | 3.0 | 3.0 | 3.0 | 3.0 | 3.0 | 3.0 |

(continued)

| Component name (% by mass) | | Example 1 | Example 2 | Example 3 | Example 4 | Example 5 | Example 6 | Example 7 | Example 8 |
|---|---|---|---|---|---|---|---|---|---|
| (G) | Ethyl[(methacryloyloxy)ethyl]dimethylammonium ethyl sulfate/N,N-dimethylacrylamide/polyethylene glycol dimethacrylate copolymer *22 | 0.1 | | 0.1 | 0.1 | 0.1 | 0.1 | 0.1 | 0.1 |
| | Poly(methacryloyloxyethyl trimethylammonium chloride) *23 | | | 0.1 | 0.4 | 0.1 | 0.1 | 0.25 | 0.1 |
| | Xanthan gum *24 | | | | | | | | |
| | Stearyl alcohol *25 | 1.5 | 1.5 | 1.5 | 1.5 | 1.5 | 1.5 | 1.5 | 1.5 |
| | Cetanol *26 | | | | | | | | |
| | Cholesterol *27 | | | | | | | | |
| | Methyl polysiloxane *28 | 1.25 | 1.25 | 1.25 | 1.25 | 1.25 | 1.25 | 1.25 | 1.25 |
| | Allantoin *29 | 0.3 | 0.3 | 0.3 | 0.3 | 0.3 | 0.3 | 0.3 | 0.3 |
| | Eucalyptus extract *30 | 0.33 | 0.33 | 0.33 | 0.33 | 0.33 | 0.33 | 0.33 | 0.33 |
| | Phenoxyethanol *31 | 0.3 | 0.3 | 0.3 | 0.3 | 0.3 | 0.3 | 0.3 | 0.3 |
| | Succinic acid *32 | 0.020 | 0.020 | 0.020 | 0.020 | 0.020 | 0.020 | 0.020 | 0.020 |
| | Arginine *33 | 0.032 | 0.032 | 0.032 | 0.032 | 0.032 | 0.032 | 0.032 | 0.032 |
| (E) | Purified water | Balance | Balance | Balance | Balance | Balance | Balance | Balance | Balance |
| Total | | 100.0 | 100.0 | 100.0 | 100.0 | 100.0 | 100.0 | 100.0 | 100.0 |
| (A) Total amount | | 5.0 | 5.0 | 5.0 | 5.0 | 3.0 | 6.5 | 5.0 | 2.0 |
| (B) Total amount | | 3.0 | 3.0 | 3.0 | 3.0 | 5.0 | 1.5 | 3.0 | 6.0 |
| (C) Total amount | | 0.80 | 0.80 | 0.80 | 0.80 | 0.80 | 0.80 | 0.80 | 0.80 |
| (D) Total amount | | 5.0 | 5.0 | 5.0 | 5.0 | 5.0 | 5.0 | 5.0 | 5.0 |
| (F) Total amount | | 0.0 | 0.0 | 0.0 | 0.0 | 0.0 | 0.0 | 0.0 | 0.0 |
| (G) Total amount | | 0.000 | 0.000 | 0.030 | 0.120 | 0.030 | 0.030 | 0.075 | 0.030 |
| (A) + (B) | | 8.0 | 8.0 | 8.0 | 8.0 | 8.0 | 8.0 | 8.0 | 8.0 |
| (A) / (B) | | 1.67 | 1.67 | 1.67 | 1.67 | 0.60 | 4.33 | 1.67 | 0.33 |
| (B) / (C) | | 3.75 | 3.75 | 3.75 | 3.75 | 6.25 | 1.88 | 3.75 | 7.50 |

(continued)

| Component name (% by mass) | Example 1 | Example 2 | Example 3 | Example 4 | Example 5 | Example 6 | Example 7 | Example 8 |
|---|---|---|---|---|---|---|---|---|
| (C) / (D) | 0.16 | 0.16 | 0.16 | 0.16 | 0.16 | 0.16 | 0.16 | 0.16 |
| (B) / (A + B) | 0.38 | 0.38 | 0.38 | 0.38 | 0.63 | 0.19 | 0.38 | 0.75 |
| Success or failure of preparation of emulsion | Good | Good | Good | Good | Good | Good | Good | Good |
| Stability after 1 week at room temperature | Good | Good | Good | Good | Good | Good | Good | Good |
| Applied to dry skin: Absorption feeling | 22 | 21 | 25 | 25 | 23 | 19 | 25 | 19 |
| Moist feeling after washing with water | 22 | 19 | 25 | 25 | 23 | 18 | 25 | 19 |
| Applied to skin wetted with water: Easy spreading | 23 | 22 | 25 | 25 | 23 | 23 | 25 | 21 |
| Absence of sliminess upon application | 24 | 25 | 25 | 25 | 24 | 22 | 25 | 21 |
| Cover or protection feeling | 22 | 18 | 25 | 25 | 23 | 17 | 25 | 19 |
| Next morning's moist feeling | 22 | 20 | 25 | 25 | 22 | 18 | 25 | 20 |
| Applied to skin wetted with water and showered off: Skin glow feeling after washing off | 22 | 18 | 25 | 25 | 23 | 18 | 25 | 19 |
| Absence of stickiness | 25 | 25 | 25 | 25 | 22 | 19 | 25 | 22 |

[Table 2]

| | Component name (% by mass) | Example 9 | Example 10 | Example 11 | Example 12 | Example 13 | Example 14 | Example 15 | Example 16 |
|---|---|---|---|---|---|---|---|---|---|
| (A) | Squalane *1 | | | | | | | | |
| | Liquid paraffin *2 | 2.0 | 2.0 | 2.0 | 5.0 | 5.0 | 5.0 | 5.0 | 5.0 |
| (B) | Di(phytosteryl/2-octyldodecyl) N-lauroyl-L-glutamate *3 | 3.0 | 3.0 | 3.0 | 3.0 | 3.0 | 3.0 | 3.0 | 3.0 |
| | Neopentyl glycol dicaprate *4 | 3.0 | | | | | | | |
| | Isononyl isononanoate *5 | | 3.0 | | | | | | |
| | Caprylic/capric triglyceride *6 | | | 3.0 | | | | | |
| (C) | Distearyl dimethylammonium chloride *7 | 0.8 | 0.8 | 0.8 | 0.8 | 0.8 | 0.8 | 0.8 | 0.8 |
| | Behentrimonium chloride *8 | | | | | | | | |
| | Sodium N-stearoyl-N-methyl taurate *9 | | | | | | | | |
| (F) | Polyoxyethylene sorbitan monostearate *10 | | | | | | | | |
| | Glyceryl behenate *11 | 0.3 | | | | | | | |
| | Sorbitan monostearate *12 | | | | | | | | |
| (D) | Petrolatum *13 | 2.0 | 2.0 | 2.0 | | | | 2.0 | 2.0 |
| | N-(Hexadesiloxyhydroxypropyl)-N-hvdroxvethylhexadecanamide *14 | 3.0 | 3.0 | 3.0 | 3.0 | 3.0 | 4.0 | 3.0 | 3.0 |
| | Cholesteryl isostearate *15 | | | | | | | | |
| | Di(phytosteryl/behenyl/2-octyldodecyl) N-lauroyl-L-glutamate *16 | | | | 2.0 | | | | |
| | Di(phytosteryl/isostearyl/cetyl/stearyl/behenyl) dilinoleate *17 | | | | | | | | 2.0 |
| | Concentrated glycerin *18 | 20.0 | 20.0 | 20.0 | 20.0 | 20.0 | 20.0 | 20.0 | 20.0 |
| | Sorbitol *19 | 8.0 | 8.0 | 8.0 | 8.0 | 8.0 | 8.0 | 8.0 | 8.0 |
| | Polyethylene glycol 600 *20 | 3.0 | 3.0 | 3.0 | 3.0 | 3.0 | 3.0 | 3.0 | 3.0 |
| (H) | d-δ-Tocopherol *21 | 0.1 | 0.1 | 0.1 | 0.1 | 0.1 | 0.1 | 0.1 | 0.1 |

(continued)

| | Component name (% by mass) | Example 9 | Example 10 | Example 11 | Example 12 | Example 13 | Example 14 | Example 15 | Example 16 |
|---|---|---|---|---|---|---|---|---|---|
| (G) | Ethyl[(methacryloyloxy)ethyl]dimethylammonium ethyl sulfate/N,N-dimethylacrylamide/polyethylene glycol dimethacrylate copolymer *22 | 0.1 | 0.1 | 0.1 | 0.1 | 0.1 | 0.1 | 0.1 | 0.1 |
| | Poly(methacryloyloxyethyl trimethylammonium chloride) *23 | | | | | | | | |
| | Xanthan gum *24 | | | | | | | | |
| | Stearyl alcohol *25 | 1.5 | 1.5 | 1.5 | 1.5 | 1.5 | 1.5 | | 1.5 |
| | Cetanol *26 | | | | | | | 1.5 | |
| | Cholesterol *27 | | | | | | | | |
| | Methyl polysiloxane *28 | 1.25 | 1.25 | 1.25 | 1.25 | 1.25 | 1.25 | 1.25 | 1.25 |
| | Allantoin *29 | 0.3 | 0.3 | 0.3 | 0.3 | 0.3 | 0.3 | 0.3 | 0.3 |
| | Eucalyptus extract *30 | 0.33 | 0.33 | 0.33 | 0.33 | 0.33 | 0.33 | 0.33 | 0.33 |
| | Phenoxyethanol *31 | 0.3 | 0.3 | 0.3 | 0.3 | 0.3 | 0.3 | 0.3 | 0.3 |
| | Succinic acid *32 | 0.020 | 0.020 | 0.020 | 0.020 | 0.020 | 0.020 | 0.020 | 0.020 |
| | Arginine *33 | 0.032 | 0.032 | 0.032 | 0.032 | 0.032 | 0.032 | 0.032 | 0.032 |
| (E) | Purified water | Balance | Balance | Balance | Balance | Balance | Balance | Balance | Balance |
| | Total | 100.0 | 100.0 | 100.0 | 100.0 | 100.0 | 100.0 | 100.0 | 100.0 |
| | (A) Total amount | 2.0 | 2.0 | 2.0 | 5.0 | 5.0 | 5.0 | 5.0 | 5.0 |
| | (B) Total amount | 6.0 | 6.0 | 6.0 | 3.0 | 3.0 | 3.0 | 3.0 | 3.0 |
| | (C) Total amount | 0.80 | 0.80 | 0.80 | 0.80 | 0.80 | 0.80 | 0.80 | 0.80 |
| | (D) Total amount | 5.0 | 5.0 | 5.0 | 5.0 | 3.0 | 4.0 | 5.0 | 7.0 |
| | (F) Total amount | 0.0 | 0.0 | 0.0 | 0.0 | 0.0 | 0.0 | 0.0 | 0.0 |
| | (G) Total amount | 0.030 | 0.030 | 0.030 | 0.030 | 0.030 | 0.030 | 0.030 | 0.030 |
| | (A) + (B) | 8.0 | 8.0 | 8.0 | 8.0 | 8.0 | 8.0 | 8.0 | 8.0 |
| | (A) / (B) | 0.33 | 0.33 | 0.33 | 1.67 | 1.67 | 1.67 | 1.67 | 1.67 |
| | (B) / (C) | 7.50 | 7.50 | 7.50 | 3.75 | 3.75 | 3.75 | 3.75 | 3.75 |

(continued)

| Component name (% by mass) | Example 9 | Example 10 | Example 11 | Example 12 | Example 13 | Example 14 | Example 15 | Example 16 |
|---|---|---|---|---|---|---|---|---|
| (C) / (D) | 0.16 | 0.16 | 0.16 | 0.16 | 0.27 | 0.20 | 0.16 | 0.11 |
| (B) / (A + B) | 0.75 | 0.75 | 0.75 | 0.38 | 0.38 | 0.38 | 0.38 | 0.38 |
| Success or failure of preparation of emulsion | Good | Good | Good | Good | Good | Good | Good | Good |
| Stability after 1 week at room temperature | Good | Good | Good | Good | Good | Good | Good | Good |
| Applied to dry skin: Absorption feeling | 16 | 19 | 19 | 25 | 23 | 24 | 25 | 22 |
| Moist feeling after washing with water | 15 | 19 | 19 | 25 | 23 | 24 | 25 | 25 |
| Applied to skin wetted with water: Easy spreading | 21 | 21 | 21 | 25 | 22 | 22 | 25 | 21 |
| Absence of sliminess upon application | 16 | 22 | 20 | 25 | 25 | 25 | 25 | 25 |
| Cover or protection feeling | 15 | 19 | 20 | 25 | 23 | 24 | 25 | 25 |
| Next morning's moist feeling | 16 | 19 | 20 | 25 | 24 | 25 | 25 | 25 |
| Applied to skin wetted with water and showered off: Skin glow feeling after washing off | 15 | 19 | 19 | 25 | 21 | 21 | 25 | 25 |
| Absence of stickiness | 16 | 23 | 20 | 25 | 24 | 25 | 25 | 21 |

[Table 3]

| Component name (% by mass) | | Example 17 | Example 18 | Example 19 | Comparative Example 1 | Comparative Example 2 | Comparative Example 3 | Comparative Example 4 | Comparative Example 5 |
|---|---|---|---|---|---|---|---|---|---|
| (A) | Squalane *1 | | | | | 3.5 | | | |
| | Liquid paraffin *2 | 5.0 | 5.0 | 5.0 | 8.0 | | 8.0 | | 5.0 |
| (B) | Di(phytosteryl/2-octyldodecyl) N-lauroyl-L-glutamate *3 | 3.0 | 3.0 | 3.0 | | | | 8.0 | 3.0 |
| | Neopentyl glycol dicaprate *4 | | | | | | | | |
| | Isononyl isononanoate *5 | | | | | | | | |
| | Caprylic/capric triglyceride *6 | | | | | | | | |
| (C) | Distearyl dimethylammonium chloride *7 | | 0.8 | 0.8 | 0.8 | | 0.8 | 0.8 | 0.8 |
| | Behentrimonium chloride *8 | 1.0 | | | | | | | |
| | Sodium N-stearoyl-N-methyl taurate *9 | | | | | 0.2 | | | |
| (F) | Polyoxyethylene sorbitan monostearate *10 | | | | | 1.6 | | | |
| | Glyceryl behenate *11 | | | | | | | | |
| | Sorbitan monostearate *12 | | | | | 0.8 | | | |
| (D) | Petrolatum *13 | 2.0 | 2.0 | 2.0 | 2.0 | | 2.0 | 2.0 | |
| | N-(Hexadesiloxyhydroxypropyl)-N-hvdroxvethylhexadecanamide *14 | 3.0 | 3.0 | 3.0 | 3.0 | 3.0 | 3.0 | 3.0 | |
| | Cholesteryl isostearate *15 | | | | | 0.3 | | | |
| | Di(phytosteryl/behenyl/2-octyldodecyl) N-lauroyl-L-glutamate *16 | | | | | | | | |
| | Di(phytosteryl/isostearyl/cetyl/stearyl/behenyl) dilinoleate *17 | | | | | | | | |
| | Concentrated glycerin *18 | 20.0 | 20.0 | 20.0 | 20.0 | 9.5 | 20.0 | 20.0 | 20.0 |
| | Sorbitol *19 | 8.0 | 8.0 | 8.0 | 8.0 | | 8.0 | 8.0 | 8.0 |
| | Polyethylene glycol 600 *20 | 3.0 | 3.0 | 3.0 | 3.0 | | 3.0 | 3.0 | 3.0 |
| (H) | d-δ-Tocopherol *21 | 0.1 | 0.1 | 0.1 | | | 0.1 | 0.1 | 0.1 |

(continued)

| | Component name (% by mass) | Example 17 | Example 18 | Example 19 | Comparative Example 1 | Comparative Example 2 | Comparative Example 3 | Comparative Example 4 | Comparative Example 5 |
|---|---|---|---|---|---|---|---|---|---|
| (G) | Ethyl[(methacryloyloxy)ethyl]dimethylammonium ethyl sulfate/N,N-dimethylacrylamide/polyethylene glycol dimethacrylate copolymer *22 | 0.1 | | 0.1 | | | 0.1 | 0.1 | 0.1 |
| | Poly(methacryloyloxyethyl trimethylammonium chloride) *23 | | 0.1 | | | | | | |
| | Xanthan gum *24 | | | | | 0.3 | | | |
| | Stearyl alcohol *25 | 0.6 | 0.6 | 0.6 | 1.5 | 0.4 | 1.5 | 1.5 | 1.5 |
| | Cetanol *26 | 0.9 | 0.9 | 0.9 | | 0.6 | | | |
| | Cholesterol *27 | | | | | 0.8 | | | |
| | Methyl polysiloxane *28 | 1.25 | 1.25 | 1.25 | 1.25 | 1.50 | 1.25 | 1.25 | 1.25 |
| | Allantoin *29 | 0.3 | 0.3 | | 0.3 | 0.3 | 0.3 | 0.3 | 0.3 |
| | Eucalyptus extract *30 | 0.33 | 0.33 | | 0.33 | 0.33 | 0.33 | 0.33 | 0.33 |
| | Phenoxyethanol *31 | 0.3 | 0.3 | | 0.3 | | 0.3 | 0.3 | 0.3 |
| | Succinic acid *32 | 0.020 | 0.020 | 0.020 | 0.020 | 0.050 | 0.020 | 0.020 | 0.020 |
| | Arginine *33 | 0.032 | 0.032 | 0.032 | 0.032 | | 0.032 | 0.032 | 0.032 |
| (E) | Purified water | Balance | Balance | Balance | Balance | Balance | Balance | Balance | Balance |
| | Total | 100.0 | 100.0 | 100.0 | 100.0 | 100.0 | 100.0 | 100.0 | 100.0 |
| | (A) Total amount | 5.0 | 5.0 | 5.0 | 8.0 | 3.5 | 8.0 | 0.0 | 5.0 |
| | (B) Total amount | 3.0 | 3.0 | 3.0 | 0.0 | 0.0 | 0.0 | 8.0 | 3.0 |
| | (C) Total amount | 0.80 | 0.80 | 0.80 | 0.80 | 0.00 | 0.80 | 0.80 | 0.80 |
| | (D) Total amount | 5.0 | 5.0 | 5.0 | 5.0 | 3.3 | 5.0 | 5.0 | 0.0 |
| | (F) Total amount | 0.0 | 0.0 | 0.0 | 0.0 | 1.6 | 0.0 | 0.0 | 0.0 |
| | (G) Total amount | 0.030 | 0.097 | 0.030 | 0.000 | 0.000 | 0.030 | 0.030 | 0.030 |
| | (A) + (B) | 8.0 | 8.0 | 8.0 | 8.0 | 3.5 | 8.0 | 8.0 | 8.0 |
| | (A) / (B) | 1.67 | 1.67 | 1.67 | - | - | - | 000 | 1.67 |
| | (B) / (C) | 3.75 | 3.75 | 3.75 | 0.00 | 0.00 | 000 | 10.00 | 3.75 |

(continued)

| Component name (% by mass) | Example 17 | Example 18 | Example 19 | Comparative Example 1 | Comparative Example 2 | Comparative Example 3 | Comparative Example 4 | Comparative Example 5 |
|---|---|---|---|---|---|---|---|---|
| (C) / (D) | 0.16 | 0.16 | 0.16 | 0.16 | - | 0.16 | 0.16 | - |
| (B) / (A + B) | 0.38 | 0.38 | 0.38 | 000 | 0.00 | 0.00 | 1.00 | 0.38 |
| Success or failure of preparation of emulsion | Good | Good | Good | Poor | Good | Poor | Poor | Good |
| Stability after 1 week at room temperature | Good | Good | Good | Poor | Good | Poor | Poor | Poor |
| Applied to dry skin: Absorption feeling | 23 | 22 | 25 | - | 14 | - | - | 11 |
| Moist feeling after washing with water | 22 | 22 | 25 | - | 6 | - | - | 10 |
| Applied to skin wetted with water: Easy spreading | 24 | 24 | 25 | - | 21 | - | - | 12 |
| Absence of sliminess upon application | 21 | 18 | 25 | - | 16 | - | - | 14 |
| Cover or protection feeling | 23 | 24 | 25 | - | 7 | - | - | 7 |
| Next morning's moist feeling | 24 | 25 | 25 | - | 5 | - | - | 8 |
| Applied to skin wetted with water and showered off: Skin glow feeling after washing off | 23 | 23 | 25 | - | 5 | - | - | 13 |
| Absence of stickiness | 21 | 18 | 25 | - | 13 | - | - | 8 |

[Table 4]

| Component name(% by mass) | | Example 20 | Example 21 | Example 22 | Example 23 | Example 24 | Comparative Example 6 | Comparative Example 7 |
|---|---|---|---|---|---|---|---|---|
| (A) | Squalane *1 | | | | | | | |
| | Liquid paraffin *2 | 5.0 | 5.0 | 5.0 | 5.0 | 5.0 | 5.0 | 5.0 |
| (B) | Di(phytosteryl/2-octyldodecyl) N-lauroyl-L-glutamate *3 | 3.0 | 3.0 | 3.0 | 3.0 | 3.0 | 3.0 | 3.0 |
| | Neopentyl glycol dicaprate *4 | | | | | | | |
| | Isononyl isononanoate *5 | | | | | | | |
| | Caprylic/capric triglyceride *6 | | | | | | | |
| (C) | Distearyl dimethylammonium chloride *7 | 0.4 | 2.0 | | 0.8 | 0.8 | 0.8 | 0.8 |
| | Behentrimonium chloride *8 | | | | | | | |
| | Lauryl hydroxysulfobetaine liquid *34 | | | 2.7 | | | | |
| | Sodium N-stearoyl-N-methyl taurate *9 | | | | | | | |
| (F) | Polyoxyethylene sorbitan monostearate *10 | | | | | | | |
| | Glyceryl behenate *11 | | | | | | | |
| | Sorbitan monostearate *12 | | | | | | | |
| (D) | Petrolatum *13 | 2.0 | 2.0 | 2.0 | 1.0 | 12.0 | 1.0 | 20.0 |
| | N-(Hexadesiloxyhydroxypropyl)-N-hydroxyethylhexadecanamide *14 | 3.0 | 3.0 | 3.0 | 1.0 | 3.0 | 0.5 | 3.0 |
| | Cholesteryl isostearate *15 | | | | | | | |
| | Di(phytosteryl/behenyl/2-octyldodecyl) N-lauroyl-L-glutamate *16 | | | | | | | |
| | Di(phytosteryl/isostearyl/cetyl/stearyl/behenyl) dilinoleate *17 | | | | | | | |
| | Concentrated glycerin *18 | 20.0 | 20.0 | 20.0 | 20.0 | 20.0 | 20.0 | 20.0 |
| | Sorbitol *19 | 8.0 | 8.0 | 8.0 | 8.0 | 8.0 | 8.0 | 8.0 |
| | Polyethylene glycol 600 *20 | 3.0 | 3.0 | 3.0 | 3.0 | 3.0 | 3.0 | 3.0 |
| (H) | d-δ-Tocopherol *21 | 0.1 | 0.1 | 0.1 | 0.1 | 0.1 | 0.1 | 0.1 |

(continued)

| | Component name(% by mass) | Example 20 | Example 21 | Example 22 | Example 23 | Example 24 | Comparative Example 6 | Comparative Example 7 |
|---|---|---|---|---|---|---|---|---|
| (G) | Ethyl[(methacryloyloxy)ethyl]dimethylammonium ethyl sulfate/N,N-dimethylacrylamide/polyethylene glycol dimethacrylate copolymer *22 | 0.1 | 0.1 | 0.1 | 0.1 | 0.1 | 0.1 | 0.1 |
| | Poly(methacryloyloxyethyl trimethylammonium chloride) *23 | | | | | | | |
| | Xanthan gum *24 | | | | | | | |
| | Stearyl alcohol *25 | 1.5 | 1.5 | 1.5 | 1.5 | 1.5 | 1.5 | 1.5 |
| | Cetanol *26 | | | | | | | |
| | Cholesterol *27 | | | | | | | |
| | Methyl polysiloxane *28 | 1.25 | 1.25 | 1.25 | 1.25 | 1.25 | 1.25 | 1.25 |
| | Allantoin *29 | 0.3 | 0.3 | 0.3 | 0.3 | 0.3 | 0.3 | 0.3 |
| | Eucalyptus extract *30 | 0.33 | 0.33 | 0.33 | 0.33 | 0.33 | 0.33 | 0.33 |
| | Phenoxyethanol *31 | 0.3 | 0.3 | 0.3 | 0.3 | 0.3 | 0.3 | 0.3 |
| | Succinic acid *32 | 0.020 | 0.020 | 0.020 | 0.020 | 0.020 | 0.020 | 0.020 |
| | Arginine *33 | 0.032 | 0.032 | 0.032 | 0.032 | 0.032 | 0.032 | 0.032 |
| (E) | Purified water | Balance | Balance | Balance | Balance | Balance | Balance | Balance |
| | Total | 100.0 | 100.0 | 100.0 | 100.0 | 100.0 | 100.0 | 100.0 |
| | (A) Total amount | 5.0 | 5.0 | 5.0 | 5.0 | 5.0 | 5.0 | 5.0 |
| | (B) Total amount | 3.0 | 3.0 | 3.0 | 3.0 | 3.0 | 3.0 | 3.0 |
| | (C) Total amount | 0.40 | 2.00 | 0.80 | 0.80 | 0.80 | 0.80 | 0.80 |
| | (D) Total amount | 5.0 | 5.0 | 5.0 | 2.0 | 15.0 | 1.5 | 23.0 |
| | (F) Total amount | 0.0 | 0.0 | 0.0 | 0.0 | 0.0 | 0.0 | 0.0 |
| | (G) Total amount | 0.030 | 0.030 | 0.030 | 0.030 | 0.030 | 0.030 | 0.030 |
| | (A) + (B) | 8.0 | 8.0 | 8.0 | 8.0 | 8.0 | 8.0 | 8.0 |
| | (A) / (B) | 1.67 | 1.67 | 1.67 | 1.67 | 1.67 | 1.67 | 1.67 |
| | (B) / (C) | 7.50 | 1.50 | 3.75 | 3.75 | 3.75 | 3.75 | 3.75 |
| | (C) / (D) | 0.08 | 0.40 | 0.16 | 0.40 | 0.05 | 0.53 | 0.03 |

(continued)

| Component name(% by mass) | Example 20 | Example 21 | Example 22 | Example 23 | Example 24 | Comparative Example 6 | Comparative Example 7 |
|---|---|---|---|---|---|---|---|
| (B) / (A + B) | 0.38 | 0.38 | 0.38 | 0.38 | 0.38 | 0.38 | 0.38 |
| Success or failure of preparation of emulsion | Good | Good | Good | Good | Good | Good | Poor |
| Stability after 1 week at room temperature | Good | Good | Good | Good | Good | Good | - |
| Applied to dry skin: Absorption feeling | 25 | 15 | 15 | 21 | 19 | 11 | - |
| Moist feeling after washing with water | 25 | 16 | 15 | 20 | 20 | 11 | - |
| Applied to skin wetted with water: Easy spreading | 25 | 18 | 18 | 20 | 20 | 14 | - |
| Absence of sliminess upon application | 25 | 16 | 17 | 22 | 18 | 14 | - |
| Cover or protection feeling | 25 | 15 | 15 | 20 | 22 | 11 | - |
| Next morning's moist feeling | 25 | 15 | 16 | 20 | 22 | 10 | - |
| Applied to skin wetted with water and showered off: Skin glow feeling after washing off | 25 | 15 | 15 | 21 | 17 | 10 | - |
| Absence of stickiness | 25 | 18 | 18 | 20 | 15 | 11 | - |

* Comparative Example 7 was not subjected to the other evaluations due to separation.
*1: Squalane: manufactured by Kishimoto Special Liver Oil Co., Ltd., Squalane,
*2: Liquid paraffin: manufactured by Sonneborn, LLC, Kaydol,
*3: Di(phytosteryl/2-octyldodecyl) N-lauroyl-L-glutamate: manufactured by Ajinomoto Co., Inc., ELDEW PS-203,
*4: Neopentyl glycol dicaprate: manufactured by The Nisshin OilliO Group, Ltd., ESTEMOL N-01,
*5: Isononyl isononanoate: manufactured by The Nisshin OilliO Group, Ltd., SALACOS 99,
*6: Caprylic/capric triglyceride: manufactured by Kao Corp., COCONARD MT,
*7: Distearyl dimethylammonium chloride: manufactured by Evonik Operations GmbH, VARISOFT TA100,
*8: Behenyl trimethylammonium chloride: manufactured by NOF Corp., CATION VB-800E (mixture of 80% behenyl trimethylammonium chloride and 20% ethanol),
*9: Sodium N-stearoyl-N-methyl taurate: manufactured by Nippon Surfactant Industries Co., Ltd., NIKKOL SMT,
*10: Polyoxyethylene sorbitan monostearate: manufactured by Kao Corp., RHEODOL TW-S120V,
*11: Glyceryl behenate: manufactured by Taiyo Kagaku Co., Ltd., SUNSOFT NO.8100-CK,
*12: Sorbitan monostearate: manufactured by Kao Corp., RHEODOL SP-S10V,
*13: Petrolatum: manufactured by Sonneborn, LLC, Super White Protopet,
*14: N-(Hexadesiloxyhydroxypropyl)-N-hydroxyethylhexadecanamide: manufactured by Kao Corp., SOFCARECERAMIDE SL-E,
*15: Cholesteryl isostearate: manufactured by Kao Corp., EXCEPARL IS-CE-A,
*16: Di(phytosteryl/behenyl/2-octyldodecyl) N-lauroyl-L-glutamate: manufactured by Ajinomoto Co., Inc., ELDEW PS-306,
*17: Di(phytosteryl/isostearyl/cetyl/stearyl/behenyl) dilinoleate: manufactured by Nippon Fine Chemical Co., Ltd., PLANDOOL-H,
*18: Concentrated glycerin: manufactured by Kao Corp., Concentrated glycerin for cosmetics,
*19: Sorbitol: manufactured by Mitsubishi Corporation Life Sciences Ltd., Sorbit W-70 (mixture of 70% sorbitol and 30% water),
*20: Polyethylene glycol 600: manufactured by Sanyo Chemical Industries, Ltd., PEG-600(-G),
*21: d-δ-Tocopherol: manufactured by Tama Biochemical Co., Ltd., D-δ-Tocopherol,
*22: Ethyl[(methacryloyloxy)ethyl]dimethylammonium ethyl sulfate/N,N-dimethylacrylamide/polyethylene glycol dimethacrylate copolymer: (mixture of 30% ethyl[(methacryloyloxy)ethyl]dimethylammonium ethyl sulfate/N,N-dimethylacrylamide and 70% polyethylene glycol),
*23: Poly(methacryloyloxyethyl trimethylammonium chloride): manufactured by BASF Personal Care and Nutrition GmbH, COSMEDIA ULTRAGEL 300 (mixture of 97% poly(methacryloyloxyethyl trimethylammonium chloride) and 3% isopropanol),
*24: Xanthan gum: manufactured by Sumitomo Pharma FOOD & CHEMICAL Co., Ltd., KELDENT,*25: Stearyl alcohol: manufactured by Kao Corp., KALCOL 8098,
*26: Cetanol: manufactured by Kao Corp., KALCOL 6098,
*27: Cholesterol: manufactured by Nippon Fine Chemical Co., Ltd., Cholesterol JSQI,
*28: Methyl polysiloxane: manufactured by Shin-Etsu Chemical Co., Ltd., Silicone KF-96A-10CS,
*29: Allantoin: manufactured by Kawaken Fine Chemicals Co., Ltd., S-Allantoin,
*30: Eucalyptus extract: manufactured by Koei Kogyo Co., Ltd., Eucalyptus Extract K,
*31: Phenoxyethanol: manufactured by TOHO Chemical Industry Co., Ltd., HISOLVE EPH,
*32: Succinic acid: manufactured by Nippon Shokubai Co., Ltd., Succinic Acid,
*33: Arginine: manufactured by Ajinomoto Co., Inc., L-Arginine C Grade
*34: Lauryl hydroxysulfobetaine liquid: manufactured by Kao Corp., AMPHITOL 20HD (mixture of 30% by mass of lauryl hydroxysulfobetaine and 70% by mass of water)

The oil-in-water-type emulsion composition of the present invention has the following feature: the oil-in-water-type emulsion composition is well sorbed or absorbed to the skin upon application to the skin and is not removed even if washed with water or toweled. Particularly, the oil components efficiently remain on the skin. Accordingly, the amount of an oil component remaining on the skin after application to wet skin and toweling was measured with ceramide taken as an example as to some of Examples and Comparative Examples. The residual ratio of the oil component (ceramide) remaining on the skin after application to wet skin and toweling correlates with a skin cover or protection feeling or next morning's moist feeling. The oil-in-water-type emulsion composition of the present invention has a high residual ratio of the oil component (ceramide) and can therefore attain a favorable skin cover or protection feeling or next morning's moist feeling.

Test Example

The residual ratio of ceramide was measured when the oil-in-water-type emulsion compositions of Examples 3 to

6 and Comparative Example 2 were each applied to the skin. The results are shown in Table 5.

(Testing method)

(1) A test site on the inner side of the forearm was washed using Curel Foam Body Wash (manufactured by Kao Corp.), which was then washed off with warm water and toweled.
(2) 82 μL of each oil-in-water-type emulsion composition was put on the thus washed test site (5 × 6 cm) on the inner side of the forearm using Microman, mixed by the addition of 153 μL of tap water, and uniformly applied thereto.
(3) After being left for 1 minute, the forearm was dried with paper towel by dab twice.
(4) The site that had received the applied oil-in-water-type emulsion composition was sampled five times in the depth direction at the same location using D-SQUAME Tape (Clinical and Derm LLC, in a circle of 2.3 cm in diameter). Another site different from the collection location was sampled five times in the same manner as above. This sampling was performed from a total of four locations (see Fig. 1).
(5) All the 20 sampled tapes were placed in a vial, to which 5 mL of acetone/diethyl ether/ethanol = 3/3/4 was then added, followed by extraction by vortex and subsequent drying. Further, 5 mL of methanol was placed in the vial, which was then capped, followed by extraction for 1.5 hours in an ultrasonic washing machine (up to 50°C). N-(Hexadesiloxyhydroxypropyl)-N-hydroxyethylhexadecanamide (component (D1)) in the extract was quantified by HPLC (UV, ODS column) using methanol/acetonitrile = 7/3.
(6) The residual ratio of ceramide was determined according to the equation given below from the amount of N-(hexadesiloxyhydroxypropyl)-N-hydroxyethylhexadecanamide (component (D1)) adhering after towel dry to the application site (remaining without being removed with paper towel), the amount (% by mass) of the component contained in the oil-in-water-type emulsion composition, and the amount of the oil-in-water-type emulsion composition applied on the basis of the analysis values.

For the calculation of the residual ratio, the recovery area (1.15 × 1.15 × 3.14 × 4 $cm^2$) based on the application area (5 × 6 $cm^2$) was taken into consideration. The calculation was performed on the assumption that the composition was uniformly applied to the application site.
Each oil-in-water-type emulsion composition was tested twice by the above procedures (1) to (6) to determine residual ratios, an average value of which was used as a measurement value.

Residual ratio of ceramide (%) = (Total amount of the component (D1) recovered from the 20 tapes)/(Content of the component (D1) in the oil-in-water-type emulsion composition applied to the recovery area of 1.15 × 1.15 × 3.14 $cm^2$) × 100

[Table 5]

| | Example 3 | Example 4 | Example 5 | Example 6 | Comparative Example 2 |
|---|---|---|---|---|---|
| Residual Ratio of ceramide (%) | 37 | 90 | 100 | 66 | 5 |

## Claims

1. An oil-in-water-type emulsion composition comprising the following components (A), (B), (C), (D), and (E):

(A) a hydrocarbon oil which is in a liquid state at 25°C,
(B) one or more members selected from the group consisting of a higher fatty acid, a higher alcohol, and an ester oil which are in a liquid state at 25°C,
(C) from 0.1 to 5% by mass of one or more members selected from the group consisting of a cationic surfactant and an amphoteric surfactant,
(D) from 2 to 20% by mass of one or more members selected from the group consisting of a ceramide, a hydrocarbon oil which is in a paste state at 25°C, and an ester oil which is in a paste state at 25°C, and
(E) water, wherein

a total content of the components (A) and (B), (A) + (B), is from 1 to 30% by mass, and

a mass ratio of the component (B) to the total amount of the components (A) and (B), (B) / ((A) + (B)), is from 0.05 to 0.9.

2. The oil-in-water-type emulsion composition according to claim 1, wherein the component (B) comprises acylamino acid diester.

3. The oil-in-water-type emulsion composition according to claim 1 or 2, wherein the component (C) is a cationic surfactant.

4. The oil-in-water-type emulsion composition according to any one of claims 1 to 3, wherein a ceramide is contained as the component (D) at 1% by mass or more in the total composition.

5. An oil-in-water-type emulsion composition comprising the following components (A), (B), (C), (D), and (E):

(A) a hydrocarbon oil which is in a liquid state at 25°C,
(B) one or more members selected from the group consisting of a higher fatty acid, a higher alcohol, and an ester oil which are in a liquid state at 25°C,
(C) from 0.1 to 5% by mass of a cationic surfactant,
(D) from 1 to 20% by mass of a ceramide, and
(E) water.

6. The oil-in-water-type emulsion composition according to any one of claims 1 to 5, further comprising (F) less than 0.1% by mass of a nonionic surfactant having HLB of 13 or more.

7. The oil-in-water-type emulsion composition according to any one of claims 1 to 6, further comprising (G) a cationic polymer.

8. The oil-in-water-type emulsion composition according to any one of claims 1 to 7, further comprising (H) one or more members selected from the group consisting of tocopherol and a derivative thereof.

9. The oil-in-water-type emulsion composition according to any one of claims 1 to 8, wherein the oil-in-water-type emulsion composition is used by application to the skin wetted with water.

10. The oil-in-water-type emulsion composition according to any one of claims 1 to 8, wherein the oil-in-water-type emulsion composition is used by application to the skin followed by rinsing with water.

Fig. 1

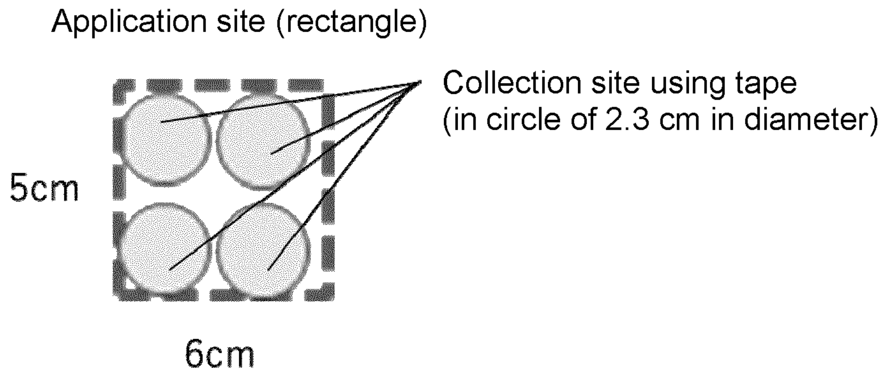
Application site (rectangle)
Collection site using tape
(in circle of 2.3 cm in diameter)
5cm
6cm

**INTERNATIONAL SEARCH REPORT**

International application No.
**PCT/JP2023/028895**

**A. CLASSIFICATION OF SUBJECT MATTER**

***A61Q 1/00***(2006.01)i; ***A61Q 19/00***(2006.01)i; ***A61K 8/06***(2006.01)i; ***A61K 8/31***(2006.01)i; ***A61K 8/34***(2006.01)i; ***A61K 8/36***(2006.01)i; ***A61K 8/37***(2006.01)i; ***A61K 8/44***(2006.01)i; ***A61K 8/67***(2006.01)i; ***A61K 8/68***(2006.01)i; ***A61K 8/81***(2006.01)i
FI: A61K8/06; A61K8/31; A61K8/44; A61K8/36; A61K8/34; A61K8/68; A61K8/37; A61Q19/00; A61Q1/00; A61K8/67; A61K8/81

According to International Patent Classification (IPC) or to both national classification and IPC

**B. FIELDS SEARCHED**

Minimum documentation searched (classification system followed by classification symbols)

A61Q1/00; A61Q19/00; A61K8/06; A61K8/31; A61K8/34; A61K8/36; A61K8/37; A61K8/44; A61K8/67; A61K8/68; A61K8/81

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X<br>A | WO 1996/003107 A1 (SHISEIDO CO., LTD.) 08 February 1996 (1996-02-08)<br>claims, p. 28, line 20 to p. 29, line 18, example 9 | 1<br>2-10 |
| A | JP 2018-008907 A (KAO CORP.) 18 January 2018 (2018-01-18)<br>entire text | 1-10 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **25 September 2023** | **10 October 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2023/028895**

| Patent document cited in search report | Publication date (day/month/year) | Patent family member(s) | Publication date (day/month/year) |
|---|---|---|---|
| WO 1996/003107 A1 | 08 February 1996 | US 5763497 A<br>claims, column 15, example 9<br>KR 10-1996-0705538 A | |
| JP 2018-008907 A | 18 January 2018 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2015030705 A
- JP 2007022997 A
- JP S62228048 A
- JP S63216812 A
- JP S63227513 A
- JP S6429347 A
- JP S6431752 A
- JP H08319263 A
- JP S53139734 A
- JP S6036407 A